# EUROPEAN PATENT APPLICATION

(11) **EP 4 357 367 A1**
(43) Date of publication of application: **24.04.2024**
(21) Application number: 23745371.7
(22) Date of filing: 01.06.2023
(51) Int. Cl.: C07K 16/46, C12N 15/13, A61K 39/395, A61P 35/00, A61P 35/02

(54) **PHARMACEUTICAL COMPOSITION AND USE THEREOF**

(30) Priority: 09.09.2022 CN 202211101982
(71) Applicant: Akeso Biopharma, Inc., Zhongshan, Guangdong 528437 (CN); Akeso Pharmaceuticals, Inc., Guangzhou, Guangdong 510799 (CN)
(72) Inventor: WANG, Zhongmin, Zhongshan Guangdong 528437 (CN); LI, Baiyong, Zhongshan Guangdong 528437 (CN); XIA, Yu, Zhongshan Guangdong 528437 (CN)
(74) Representative: Cooley (UK) LLP
(86) International application number: PCT/CN2023/097757
(87) International publication number: WO 2024/051223

(57) **Abstract**

The present invention belongs to the field of tumor treatment and molecular immunology, and specifically relates to a therapeutic combination and use thereof. Specifically, the therapeutic combination comprises an anti-CTLA4-anti-PD-1 bispecific antibody and an anti-PD-1-anti-VEGFA bispecific antibody. The therapeutic combination of the present invention can effectively treat or prevent a tumor and has good application prospects.

## Description

### TECHNICAL FIELD

The present invention belongs to the field of tumor treatment and molecular immunology, and specifically relates to a therapeutic combination and use thereof. Specifically, the therapeutic combination is a pharmaceutical composition or a combination product. Specifically, the pharmaceutical composition or combination product comprises an anti-CTLA4-anti-PD-1 bispecific antibody or an antigen-binding fragment thereof, and an anti-PD-1-anti-VEGFA bispecific antibody or an antigen-binding fragment thereof.

### BACKGROUND

The transmembrane receptor PD-1 (programmed cell death protein-1) is a member of the CD28 family, and is expressed in activated T cells, B cells, and myeloid cells. Both ligands of PD-1, PDL1 (programmed cell death 1 ligand 1, or PD-L1) and PDL2 (programmed cell death 1 ligand 2, or PD-L2), are members of the B7 superfamily, wherein PDL1 is expressed in a variety of cells including T cells, B cells, endothelial cells, and epithelial cells, and PDL2 is expressed only in antigen-presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate the tumor cell killing process, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3):466-473), leukemia and anemia (Held SA, Heine A, et al., 2013, Curr Cancer Drug Targets., 13(7):768-74).

Cytotoxic T lymphocyte-associated antigen 4 (CTLA4) and CD28 molecules are very similar in aspects of gene structure, chromosome location, sequence homology and gene expression. Both molecules are receptors of co-stimulatory molecule B7, and mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5).

Interleukin 2 (IL-2) is produced by T cells. It is a growth factor that regulates T cell subgroups, and an important factor in regulating immune responses. It promotes the proliferation of activated B cells, and participates in antibody responses, hematopoiesis and tumor surveillance. Recombinant human IL-2 has been approved by the U.S. FDA for treating malignant tumors, including melanoma, renal tumor, etc., while a clinical study is currently ongoing for treating chronic viral infections (Chavez, A.R., et al., 2009, Ann. N. Y. Acad Sci., 1182:p.14-27). CTLA4 and CTLA4 antibodies are important influencing factors of T cell functions and interfere with the immune microenvironment in the body. *In-vitro* and *in-vivo* studies demonstrated that CTLA4 antibodies can specifically relieve the immunosuppression of CTLA4, activate T cells, and induce IL-2 generation, and are promising in wide applications in gene therapy against diseases such as tumors and parasite infections.

CTLA4 antibodies can produce specific therapeutic effect on diseases and show remarkable efficacy, and may be used for supplementing traditional medicines and for exploring new means of gene therapy.

Bispecific antibodies, also known as bifunctional antibodies, are specific drugs that target two different antigens simultaneously, and can be produced by immunomagnetic separation. Alternatively, they can be obtained by genetic engineering. The genetic engineering has flexibility in aspects of binding site optimization, synthetic form, yield and the like, thus having certain advantages. Currently, over 45 forms have been demonstrated (Dafne Muller, Kontermann R E., 2010, BioDrugs, 24(2):89-98). A number of developed bispecific antibodies are in the form of IgG-scFv, i.e., the Morrison format (Coloma MJ, Morrison SL., 1997, Nat Biotechnol., 15:159-163), which has been demonstrated to be one of the ideal forms for the bifunctional antibodies because of its similarity to the naturally existing IgG forms and advantages in antibody engineering, expression and purification (Miller BR, Demarest SJ, et al., 2010, Protein Eng Des Sel, 23:549-57; Fitzgerald J, Lugovskoy A., 2011, MAbs, 3:299-309).

Vascular endothelial growth factor (VEGF) is a growth factor that can promote the division and proliferation of endothelial cells, promote the formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of the tumor, improve vascular permeability, cause fibrin deposition in surrounding tissues, and promote infiltration of mononuclear cells, fibroblasts and endothelial cells, which facilitates the formation of tumor stroma and entry of tumor cells into new blood vessels, and promote tumor metastasis. Therefore, inhibiting tumor angiogenesis is considered as one of the most promising tumor treatment methods at present. The VEGF family includes: VEGFA, VEGFB, VEGFC, VEGFD and PIGF. Vascular endothelial growth factor receptors (VEGFRs) include VEGFR1 (also known as Flt1), VEGFR2 (also known as KDR or Flk1), VEGFR3 (also known as Flt4) and Neuropilin-1 (NRP-1). The first three receptors are members of the tyrosine kinase superfamily, and are of similar structures composed of an extramembrane region, a transmembrane segment and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors. VEGFA mainly acts in combination with VEGFR1, VEGFR2 and NRP-1. VEGFR1 is the first identified receptor, and has a higher affinity for VEGFA than VEGFR2 under normal physiological conditions but a lower tyrosinase activity in the intracellular segment than VEGFR2 (Ma Li, Chinese Journal of Birth Health and Heredity, 24(5) (2016):146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and other progresses of vascular endothelial cells, as well as the formation process of blood vessels and the permeability of blood vessels (Roskoski R Jr. et al., Crit Rev Oncol Hematol, 62(3) (2007):179-213). VEGFA, after binding to VEGFR2, mediates the transcription and expression of related intracellular protein genes through the downstream PLC-γ-PKC-Raf-MEK-MAPK signaling pathway, and thus promotes the proliferation of vascular endothelial cells (Takahashi T et al., Oncogene, 18(13) (1999):2221-2230).

VEGFR3 is one of the tyrosine kinase family members, and is mainly expressed in embryonic vascular endothelial cells and mature lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein, and is incapable of independently transducing biological signals but able to mediate signaling only after forming a complex with a VEGF tyrosine kinase receptor. (Ma Li, Chinese Journal of Birth Health and Heredity, 24(5) (2016): 146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase, infiltration to peripheral tissues and other patterns of endothelial cells (Dong Hongchao et al., Sep. 2014, Journal of Modern Oncology, 22(9): 2231-3).

For many cancer patients, the disease is still uncontrollable for a long term after surgery or chemotherapy, and the 5-year survival rate is still very low. In summary, developing a treatment mean or combination administration regimen with lower toxicity and higher efficacy is clinically of great meaning.

### SUMMARY

The inventors of the present invention have made intensive studies and creative efforts to combine the anti-CTLA4-anti-PD-1 bispecific antibody with the anti-PD-1-anti-VEGFA bispecific antibody, and surprisingly found that the combined antibody has a pharmacological effect of effectively inhibiting tumor growth, which is superior to that of the anti-VEGFA-anti-PD-1 bispecific antibody or the anti-CTLA4-anti-PD-1 bispecific antibody alone. The present invention is detailed below.

One aspect of the present invention relates to a therapeutic combination comprising an effective amount of a first bispecific antibody and an effective amount of a second bispecific antibody, wherein:
(1) the first bispecific antibody comprises:
   a first protein functional region targeting PD-1, and
   a second protein functional region targeting CTLA4;
   wherein the first protein functional region is an immunoglobulin, and the second protein functional region is a single chain antibody; or, the first protein functional region is a single chain antibody, and the second protein functional region is an immunoglobulin;
   wherein,
   a heavy chain variable region of the immunoglobulin comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively; a heavy chain variable region of the single chain antibody comprises HCDR1-HCDR3 having amino acid sequences set forth in SEQ ID NOs: 33-35 respectively, and a light chain variable region thereof comprises LCDR1-LCDR3 having amino acid sequences set forth in SEQ ID NOs: 36-38 respectively;
      or,
   a heavy chain variable region of the immunoglobulin comprises HCDR1-HCDR3 having amino acid sequences set forth in SEQ ID NOs: 33-35 respectively, and a light chain variable region thereof comprises LCDR1-LCDR3 having amino acid sequences set forth in SEQ ID NOs: 36-38 respectively; a heavy chain variable region of the single chain antibody comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively;
(2) the second bispecific antibody comprises:
   a first protein functional region targeting PD-1, and
   a second protein functional region targeting VEGFA;
   wherein the first protein functional region is an immunoglobulin, and the second protein functional region is a single chain antibody; or, the first protein functional region is a single chain antibody, and the second protein functional region is an immunoglobulin;
   wherein,
   a heavy chain variable region of the immunoglobulin comprises HCDR1-HCDR3 having amino acid sequences set forth in SEQ ID NOs: 58-60 respectively, and a light chain variable region thereof comprises LCDR1-LCDR3 having amino acid sequences set forth in SEQ ID NOs: 61-63 respectively; a heavy chain variable region of the single chain antibody comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively;
      or,
   a heavy chain variable region of the immunoglobulin comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively; a heavy chain variable region of the single chain antibody comprises HCDR1-HCDR3 having amino acid sequences set forth in SEQ ID NOs: 58-60 respectively, and a light chain variable region thereof comprises LCDR1-LCDR3 having amino acid sequences set forth in SEQ ID NOs: 61-63 respectively.

In some embodiments of the present invention, for the therapeutic combination,
in the (1), the immunoglobulin is of human IgG1 subtype; preferably, according to the EU numbering system, the heavy chain constant region of the immunoglobulin has one of the following combinations of mutations:
L234A and L235A; or
L234A and G237A; or
L235A and G237A; or
L234A, L235A and G237A.

In some embodiments of the present invention, for the therapeutic combination,
in the (2), the immunoglobulin is of human IgG1 subtype; preferably, according to the EU numbering system, the heavy chain constant region of the immunoglobulin has one of the following combinations of mutations:
L234A and L235A; or
L234A and G237A; or
L235A and G237A; or
L234A, L235A and G237A.

In some embodiments of the present invention, for the therapeutic combination,
in the (1), the immunoglobulin is of human IgG1 subtype; preferably, according to the EU numbering system, the heavy chain constant region of the immunoglobulin has one of the following combinations of mutations:
   L234A and L235A; or
   L234A and G237A; or
   L235A and G237A; or
   L234A, L235A and G237A;
      and
in the (2), the immunoglobulin is of human IgG1 subtype, wherein according to the EU numbering system, the heavy chain constant region of the immunoglobulin has one of the following combinations of mutations:
   L234A and L235A; or
   L234A and G237A; or
   L235A and G237A; or
   L234A, L235A and G237A.

In some embodiments of the present invention, for the therapeutic combination, wherein in the (1):
an amino acid sequence of the heavy chain variable region of the immunoglobulin is selected from SEQ ID NO: 14 and SEQ ID NO: 18; an amino acid sequence of the light chain variable region of the immunoglobulin is selected from SEQ ID NO: 16 and SEQ ID NO: 20; an amino acid sequence of the heavy chain variable region of the single chain antibody is selected from SEQ ID NO: 2, SEQ ID NO: 6, SEQ ID NO: 10, SEQ ID NO: 41 and SEQ ID NO: 43; an amino acid sequence of the light chain variable region of the single chain antibody is selected from SEQ ID NO: 4, SEQ ID NO: 8, SEQ ID NO: 12, SEQ ID NO: 42 and SEQ ID NO: 44;
   or,
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43; an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain antibody is selected from SEQ ID NO: 14 and SEQ ID NO: 18; an amino acid sequence of the light chain variable region of the single chain antibody is selected from SEQ ID NO: 16 and SEQ ID NO: 20.

In some embodiments of the present invention, for the therapeutic combination, wherein the characteristics of the (1) are selected from any of the following 1) to 20):
1) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 2, and the light chain variable region of the single chain antibody has an amino acid sequence set forth in SEQ ID NO: 4;
2) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 6, and the light chain variable region of the single chain antibody has an amino acid sequence set forth in SEQ ID NO: 8;
3) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 10, and the light chain variable region of the single chain antibody has an amino acid sequence set forth in SEQ ID NO: 12;
4) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 2, and the light chain variable region of the single chain antibody has an amino acid sequence set forth in SEQ ID NO: 4;
5) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 6, and the light chain variable region of the single chain antibody has an amino acid sequence set forth in SEQ ID NO: 8;
6) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 10, and the light chain variable region of the single chain antibody has an amino acid sequence set forth in SEQ ID NO: 12;
7) the heavy chain variable region of the immunoglobulin has an amino acid sequence set forth in SEQ ID NO: 2, and the light chain variable region of the immunoglobulin has an amino acid sequence set forth in SEQ ID NO: 4; the heavy chain variable region of the single chain antibody has an amino acid sequence set forth in SEQ ID NO: 14, and the light chain variable region of the single chain antibody has an amino acid sequence set forth in SEQ ID NO: 16;
8) the heavy chain variable region of the immunoglobulin has an amino acid sequence set forth in SEQ ID NO: 2, and the light chain variable region of the immunoglobulin has an amino acid sequence set forth in SEQ ID NO: 4; the heavy chain variable region of the single chain antibody has an amino acid sequence set forth in SEQ ID NO: 18, and the light chain variable region of the single chain antibody has an amino acid sequence set forth in SEQ ID NO: 20;
9) the heavy chain variable region of the immunoglobulin has an amino acid sequence set forth in SEQ ID NO: 6, and the light chain variable region of the immunoglobulin has an amino acid sequence set forth in SEQ ID NO: 8; the heavy chain variable region of the single chain antibody has an amino acid sequence set forth in SEQ ID NO: 14, and the light chain variable region of the single chain antibody has an amino acid sequence set forth in SEQ ID NO: 16;
10) the heavy chain variable region of the immunoglobulin has an amino acid sequence set forth in SEQ ID NO: 6, and the light chain variable region of the immunoglobulin has an amino acid sequence set forth in SEQ ID NO: 8; the heavy chain variable region of the single chain antibody has an amino acid sequence set forth in SEQ ID NO: 18, and the light chain variable region of the single chain antibody has an amino acid sequence set forth in SEQ ID NO: 20;
11) the heavy chain variable region of the immunoglobulin has an amino acid sequence set forth in SEQ ID NO: 10, and the light chain variable region of the immunoglobulin has an amino acid sequence set forth in SEQ ID NO: 12; the heavy chain variable region of the single chain antibody has an amino acid sequence set forth in SEQ ID NO: 14, and the light chain variable region of the single chain antibody has an amino acid sequence set forth in SEQ ID NO: 16;
12) the heavy chain variable region of the immunoglobulin has an amino acid sequence set forth in SEQ ID NO: 10, and the light chain variable region of the immunoglobulin has an amino acid sequence set forth in SEQ ID NO: 12; the heavy chain variable region of the single chain antibody has an amino acid sequence set forth in SEQ ID NO: 18, and the light chain variable region of the single chain antibody has an amino acid sequence set forth in SEQ ID NO: 20;
13) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 41, and the light chain variable region of the single chain antibody has an amino acid sequence set forth in SEQ ID NO: 42;
14) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 43, and the light chain variable region of the single chain antibody has an amino acid sequence set forth in SEQ ID NO: 44;
15) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 41, and the light chain variable region of the single chain antibody has an amino acid sequence set forth in SEQ ID NO: 42;
16) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 43, and the light chain variable region of the single chain antibody has an amino acid sequence set forth in SEQ ID NO: 44;
17) the heavy chain variable region of the immunoglobulin has an amino acid sequence set forth in SEQ ID NO: 41, and the light chain variable region of the immunoglobulin has an amino acid sequence set forth in SEQ ID NO: 42; the heavy chain variable region of the single chain antibody has an amino acid sequence set forth in SEQ ID NO: 14, and the light chain variable region of the single chain antibody has an amino acid sequence set forth in SEQ ID NO: 16;
18) the heavy chain variable region of the immunoglobulin has an amino acid sequence set forth in SEQ ID NO: 43, and the light chain variable region of the immunoglobulin has an amino acid sequence set forth in SEQ ID NO: 44; the heavy chain variable region of the single chain antibody has an amino acid sequence set forth in SEQ ID NO: 14, and the light chain variable region of the single chain antibody has an amino acid sequence set forth in SEQ ID NO: 16;
19) the heavy chain variable region of the immunoglobulin has an amino acid sequence set forth in SEQ ID NO: 41, and the light chain variable region of the immunoglobulin has an amino acid sequence set forth in SEQ ID NO: 42; the heavy chain variable region of the single chain antibody has an amino acid sequence set forth in SEQ ID NO: 18, and the light chain variable region of the single chain antibody has an amino acid sequence set forth in SEQ ID NO: 20;
   and,
20) the heavy chain variable region of the immunoglobulin has an amino acid sequence set forth in SEQ ID NO: 43, and the light chain variable region of the immunoglobulin has an amino acid sequence set forth in SEQ ID NO: 44; the heavy chain variable region of the single chain antibody has an amino acid sequence set forth in SEQ ID NO: 18, and the light chain variable region of the single chain antibody has an amino acid sequence set forth in SEQ ID NO: 20.

In some embodiments of the present invention, for the therapeutic combination, wherein in the (1):
the immunoglobulin comprises a heavy chain having an amino acid sequence set forth in SEQ ID NO: 40, and a light chain having an amino acid sequence set forth in SEQ ID NO: 24.

In some embodiments of the present invention, for the therapeutic combination, wherein in the (1):
the first bispecific antibody comprises:
a first protein functional region targeting PD-1, and
a second protein functional region targeting CTLA4;
the number of the first protein functional region is 1, and the number of the second protein functional region is 2;
   wherein the first protein functional region is an immunoglobulin, and the second protein functional region is a single chain antibody;
   the immunoglobulin comprises a heavy chain having an amino acid sequence set forth in SEQ ID NO: 40, and a light chain having an amino acid sequence set forth in SEQ ID NO: 24;
   the single chain antibody comprises a heavy chain variable region having an amino acid sequence set forth in SEQ ID NO: 43, and a light chain variable region having an amino acid sequence set forth in SEQ ID NO: 44;
   the single chain antibody is linked to the C terminus or the N terminus of the heavy chain of the immunoglobulin;
   the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different; preferably, the first linker fragment and the second linker fragment each have an amino acid sequence independently selected from SEQ ID NO: 25 and SEQ ID NO: 26;
   preferably, amino acid sequences of the first linker fragment and the second linker fragment are set forth in SEQ ID NO: 26.

In some embodiments of the present invention, for the therapeutic combination, wherein in the (2):
an amino acid sequence of the heavy chain variable region of the immunoglobulin is set forth in SEQ ID NO: 45, and an amino acid sequence of the light chain variable region of the immunoglobulin is set forth in SEQ ID NO: 47; an amino acid sequence of the heavy chain variable region of the single chain antibody is selected from SEQ ID NO: 14 and SEQ ID NO: 18, and an amino acid sequence of the light chain variable region of the single chain antibody is selected from SEQ ID NO: 16, SEQ ID NO: 20 and SEQ ID NO: 49;
   or,
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16, SEQ ID NO: 20 and SEQ ID NO: 49; an amino acid sequence of the heavy chain variable region of the single chain antibody is set forth in SEQ ID NO: 45, and an amino acid sequence of the light chain variable region of the single chain antibody is set forth in SEQ ID NO: 47.

In some embodiments of the present invention, for the therapeutic combination, wherein the characteristics of the (2) are selected from any of the following 1) to 12):
1) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 16;
2) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 20;
3) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 49;
4) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 16;
5) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 20;
6) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 49;
7) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 45, and the light chain variable region of the single chain antibody has an amino acid sequence set forth in SEQ ID NO: 47;
8) the heavy chain variable region of the immunoglobulin has an amino acid sequence set forth in SEQ ID NO: 14, and the light chain variable region of the immunoglobulin has an amino acid sequence set forth in SEQ ID NO: 20; the heavy chain variable region of the single chain antibody has an amino acid sequence set forth in SEQ ID NO: 45, and the light chain variable region of the single chain antibody has an amino acid sequence set forth in SEQ ID NO: 47;
9) the heavy chain variable region of the immunoglobulin has an amino acid sequence set forth in SEQ ID NO: 14, and the light chain variable region of the immunoglobulin has an amino acid sequence set forth in SEQ ID NO: 49; the heavy chain variable region of the single chain antibody has an amino acid sequence set forth in SEQ ID NO: 45, and the light chain variable region of the single chain antibody has an amino acid sequence set forth in SEQ ID NO: 47;
10) the heavy chain variable region of the immunoglobulin has an amino acid sequence set forth in SEQ ID NO: 18, and the light chain variable region of the immunoglobulin has an amino acid sequence set forth in SEQ ID NO: 16; the heavy chain variable region of the single chain antibody has an amino acid sequence set forth in SEQ ID NO: 45, and the light chain variable region of the single chain antibody has an amino acid sequence set forth in SEQ ID NO: 47;
11) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 45, and the light chain variable region of the single chain antibody has an amino acid sequence set forth in SEQ ID NO: 47; and
12) the heavy chain variable region of the immunoglobulin has an amino acid sequence set forth in SEQ ID NO: 18, and the light chain variable region of the immunoglobulin has an amino acid sequence set forth in SEQ ID NO: 49; the heavy chain variable region of the single chain antibody has an amino acid sequence set forth in SEQ ID NO: 45, and the light chain variable region of the single chain antibody has an amino acid sequence set forth in SEQ ID NO: 47.

In some embodiments of the present invention, for the therapeutic combination, wherein in the (2):
the immunoglobulin comprises a heavy chain having an amino acid sequence set forth in SEQ ID NO: 54, and a light chain having an amino acid sequence set forth in SEQ ID NO: 56.

In some embodiments of the present invention, for the therapeutic combination, wherein in the (2):
the second bispecific antibody comprises:
a first protein functional region targeting PD-1, and
a second protein functional region targeting VEGFA;
the number of the first protein functional region is 1, and the number of the second protein functional region is 2;
wherein the first protein functional region is an immunoglobulin, and the second protein functional region is a single chain antibody;
the immunoglobulin comprises a heavy chain having an amino acid sequence set forth in SEQ ID NO: 54, and a light chain having an amino acid sequence set forth in SEQ ID NO: 56;
the single chain antibody comprises a heavy chain variable region having an amino acid sequence set forth in SEQ ID NO: 18, and a light chain variable region having an amino acid sequence set forth in SEQ ID NO: 49;
the single chain antibody is linked to the C terminus or the N terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different; preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

In some embodiments of the present invention, for the therapeutic combination, wherein in the (1) and/or the (2):
the first protein functional region is linked to the second protein functional region either directly or via a linker fragment; and/or the heavy chain variable region of the single chain antibody is linked to the light chain variable region of the single chain antibody either directly or via a linker fragment.

In some embodiments of the present invention, for the therapeutic combination, wherein in the (1) and/or the (2):
the linker fragment is (GGGGS)n, wherein n is a positive integer, preferably, n is 1, 2, 3, 4, 5 or 6.

In some embodiments of the present invention, for the therapeutic combination, wherein in the (1) and/or the (2):
the numbers of the first protein functional region and the second protein functional region are each independently 1, 2 or more.

In some embodiments of the present invention, for the therapeutic combination, wherein in the (1) and/or the (2):
the single chain antibody is linked to the C terminus or the N terminus of the heavy chain of the immunoglobulin.

In some embodiments of the present invention, for the therapeutic combination, wherein the first bispecific antibody and the second bispecific antibody are mixed together, and the therapeutic combination is a pharmaceutical composition.

In some embodiments of the present invention, for the therapeutic combination, wherein the first bispecific antibody and the second bispecific antibody are in separate packages, and the therapeutic combination is a combination product.

In some embodiments of the present invention, for the therapeutic combination, wherein the mass ratio of the first bispecific antibody to the second bispecific antibody is (10:1) to (1:5), preferably (5:1) to (1:3) or (3:1) to (1:2), and more preferably 2:1.

In some embodiments of the present invention, for the therapeutic combination, wherein the molar ratio of the first bispecific antibody to the second bispecific antibody is (10:1) to (1:10), preferably (5:1) to (1:5), (3:1) to (1:3) or (2:1) to (1:2), and more preferably 1:1.

In some embodiments of the present invention, for the therapeutic combination, wherein the unit dose of the first bispecific antibody and the second bispecific antibody is independently 100 mg to 2500 mg, 100 mg to 2000 mg, 100 mg to 1500 mg, 100 mg to 1200 mg, 100 mg to 1000 mg, 200 mg to 800 mg, 200 mg to 500 mg, 300 mg to 600 mg, 400 mg to 500 mg, or 450 mg, based on the mass of the first bispecific antibody or the second bispecific antibody.

In some embodiments of the present invention, the therapeutic combination further comprises an effective amount of an anti-tumor chemotherapeutic drug, such as an alkylating agent, an antimetabolite, an anti-tumor antibiotic, a plant-based anticancer agent, a hormone, or an immunological agent and the like.

In some embodiments of the present invention, the first bispecific antibody and the second bispecific antibody are active pharmaceutical ingredients (APIs) or active ingredients.

In some embodiments of the present invention, the therapeutic combination consists of the first bispecific antibody, the second bispecific antibody and a pharmaceutically acceptable adjuvant.

In some embodiments of the present invention, the first bispecific antibody, the second bispecific antibody and the anti-tumor chemotherapeutic drug are active pharmaceutical ingredients (APIs) or active ingredients.

In some embodiments of the present invention, the therapeutic combination consists of the first bispecific antibody, the second bispecific antibody, the anti-tumor chemotherapeutic drug and a pharmaceutically acceptable adjuvant.

In some embodiments of the present invention, the therapeutic combination further comprises one or more pharmaceutically acceptable adjuvants; preferably, further comprises a package insert.

Yet another aspect of the present invention relates to use of the therapeutic combination according to any of the aspects of the present invention for preparing a medicament for the treatment or prevention of a tumor;
preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer; preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype; preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

The therapeutic combination according to any of the aspects of the present invention for use in the treatment or prevention of a tumor;
preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype;
preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

Yet another aspect of the present invention relates to a method for treating or preventing a tumor, comprising administering to a subject in need thereof an effective amount of the therapeutic combination according to any of the aspects of the present invention; wherein
preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype;
preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

Still another aspect of the present invention relates to a unit formulation, preferably used for treating a tumor, and comprising 1-10000 mg (preferably 10-1000 mg, preferably 50-500 mg, 100-400 mg, 150-300 mg, 150-250 mg or 200 mg) of the anti-CTLA4-anti-PD-1 bispecific antibody according to any of the aspects of the present invention and 1-10000 mg (preferably 1-1000 mg, preferably 50-500 mg, 100-400 mg, 150-300 mg, 150-250 mg, 200 mg or 100 mg) of the anti-PD-1-anti-VEGFA specific antibody according to any of the aspects of the present invention, and optionally one or more of the chemotherapeutic drugs (such as platinum-based drug and/or a fluorouracil antineoplastic agent) according to the present invention; wherein the anti-CTLA4-anti-PD-1 bispecific antibody, the anti-PD-1-anti-VEGFA bispecific antibody and the chemotherapeutic drug are packaged separately.

The present invention relates to a method for preventing or treating cancer or a tumor, wherein the method comprises administering a subject in need thereof one or more unit formulations according to the present invention, preferably the anti-PD-1-anti-VEGFA bispecific antibody, the anti-CTLA4-anti-PD-1 bispecific antibody and the chemotherapeutic drug are each administered separately.

Still another aspect of the present invention relates to a single dose unit, preferably used for treating a tumor, and comprising 0.1-10000 mg (preferably 1-1000 mg, preferably 50-500 mg, 100-400 mg, 150-300 mg, 150-250 mg, 200 mg or 100 mg) of the anti-CTLA4-anti-PD-1 bispecific antibody according to any of the aspects of the present invention and 0.1-10000 mg (preferably 1-1000 mg, preferably 50-500 mg, 100-400 mg, 150-300 mg, 150-250 mg, 200 mg or 100 mg) of the anti-PD-1-anti-VEGFA bispecific antibody according to any of the aspects of the present invention.

In one or more embodiments of the present invention, the anti-CTLA4-anti-PD-1 bispecific antibody, the VEGFA-anti-PD-1 bispecific antibody and/or the chemotherapeutic drug is in a form suitable for intravenous injection or intravenous drip infusion, preferably in a liquid form.

In one or more embodiments of the present invention, the step in which an effective amount of the anti-CTLA4-anti-PD-1 bispecific antibody according to any of the aspects of the present invention and/or the anti-PD-1-anti-VEGFA bispecific antibody according to any of the aspects of the present invention is administered to a subject is before or after a surgical treatment and/or before or after a radiotherapy.

In one or more embodiments of the present invention, the unit dose of the anti-CTLA4-anti-PD-1 bispecific antibody according to any of the aspects of the present invention and/or the anti-PD-1-anti-VEGFA bispecific antibody according to any of the aspects of the present invention is 0.1-100 mg, preferably 1-10 mg per kg body weight; alternatively, the unit dose of the anti-CTLA4-anti-PD-1 bispecific antibody according to any of the aspects of the present invention and/or the anti-PD-1-anti-VEGFA bispecific antibody according to any of the aspects of the present invention is 10-1000 mg, preferably 50-500 mg, 100-400 mg, 150-300 mg, 150-250 mg or 200 mg in each subject;
preferably, the dose is given from twice daily to about once every other day, or once every 3 days, 4 days, 5 days, 6 days, 10 days, 1 week, 2 weeks, 3 weeks, 4 weeks, 5 weeks or 6 weeks;
preferably, the route of administration is intravenous drip infusion, intravenous injection, subcutaneous injection or intraperitoneal injection.

MSI refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L) and microsatellite stability (MSS). The major cause of MSI is DNA mismatch repair (MMR) deficiency. Human mismatch repair genes (MMR genes) can express corresponding mismatch repair proteins through transcription and translation. Absence of any MMR protein may lead to mismatch repair deficiency, and base pair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189:45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient mismatch repair (pMMR). The detection of dMMR is to perform an immunohistochemical assay of protein expression for four mismatch genes of MSH2, MLH1, MSH6 and PMS2 based on tumor specimens (including surgical specimens and aspiration specimens). Absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for PCR assay, inconsistency in ≥30% loci is defined as MSI-H, consistency in all loci is defined as MSS, and inconsistency between 0 and 30% is defined as MSI-L.

In one or more embodiments of the present invention, the bispecific antibody is in the form of IgG-scFv, i.e., the Morrison format.

In one or more embodiments of the present invention, for the bispecific antibody, the single chain antibody is linked to the C terminus of the heavy chain of the immunoglobulin. Since an immunoglobulin has two heavy chains, two single chain antibody molecules are linked to one immunoglobulin molecule. Preferably, the two single chain antibody molecules are identical. Preferably, the single chain antibody is linked to the C terminus of the heavy chain of the immunoglobulin by forming an amide bond via the aforementioned linker fragment.

In one or more embodiments of the present invention, the constant regions of the immunoglobulin are humanized. For example, the heavy chain constant region is Ig gamma-1 chain C region, ACCESSION: P01857, and the light chain constant region is Ig kappa chain C region, ACCESSION: P01834.

In one or more embodiments of the present invention, the bispecific antibody is a monoclonal antibody.

In one or more embodiments of the present invention, the bispecific antibody is a humanized antibody.

Antibody drugs, especially monoclonal antibodies (mAbs), have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).

The amino acid sequences of the CDRs of the monoclonal antibody in (1) to (7) below are analyzed by technical means well known to those skilled in the art, and the results are as follows:

### (1)14C12

The heavy chain variable region has an amino acid sequence set forth in SEQ ID NO: 14, and the light chain variable region has an amino acid sequence set forth in SEQ ID NO: 16.

The 3 CDRs of the heavy chain variable region have the following amino acid sequences:
HCDR1: GFAFSSYD (SEQ ID NO: 27)
HCDR2: ISGGGRYT (SEQ ID NO: 28)
HCDR3: ANRYGEAWFAY (SEQ ID NO: 29)

The 3 CDRs of the light chain variable region have the following amino acid sequences:
LCDR1: QDINTY (SEQ ID NO: 30)
LCDR2: RAN (SEQ ID NO: 31)
LCDR3: LQYDEFPLT (SEQ ID NO: 32)

### (2)14C12H1L1

The heavy chain variable region has an amino acid sequence set forth in SEQ ID NO: 18, and the light chain variable region has an amino acid sequence set forth in SEQ ID NO: 20.

The amino acid sequences of the 3 CDRs of the heavy chain variable region are identical to 14C12.

The amino acid sequences of the 3 CDRs of the light chain variable region are identical to 14C12.

### (3) 14C12H1L1(M)

The heavy chain variable region has an amino acid sequence set forth in SEQ ID NO: 18, and the light chain variable region has an amino acid sequence set forth in SEQ ID NO: 49.

The amino acid sequences of the 3 CDRs of the heavy chain variable region are identical to 14C12.

The amino acid sequences of the 3 CDRs of the light chain variable region are identical to 14C12.

### (4)4G10

The heavy chain variable region has an amino acid sequence set forth in SEQ ID NO: 2, and the light chain variable region has an amino acid sequence set forth in SEQ ID NO: 4;

The 3 CDRs of the heavy chain variable region have the following amino acid sequences:
HCDR1: GYSFTGYT (SEQ ID NO: 33)
HCDR2: INPYNNIT (SEQ ID NO: 34)
HCDR3: ARLDYRSY (SEQ ID NO: 35)

The 3 CDRs of the light chain variable region have the following amino acid sequences:
LCDR1: TGAVTTSNF (SEQ ID NO: 36)
LCDR2: GTN (SEQ ID NO: 37)
LCDR3: ALWYSNHWV (SEQ ID NO: 38)

### (5)4G10H1L1

The heavy chain variable region has an amino acid sequence set forth in SEQ ID NO: 6, and the light chain variable region has an amino acid sequence set forth in SEQ ID NO: 8;
The amino acid sequences of the 3 CDRs of the heavy chain variable region are identical to 4G10.

The amino acid sequences of the 3 CDRs of the light chain variable region are identical to 4G10.

### (6)4G10H3L3

The heavy chain variable region has an amino acid sequence set forth in SEQ ID NO: 10, and the light chain variable region has an amino acid sequence set forth in SEQ ID NO: 12;
The amino acid sequences of the 3 CDRs of the heavy chain variable region are identical to 4G10.

The amino acid sequences of the 3 CDRs of the light chain variable region are identical to 4G10.

### (7)Bevacizumab

The heavy chain variable region has an amino acid sequence set forth in SEQ ID NO: 45, and the light chain variable region has an amino acid sequence set forth in SEQ ID NO: 47;
The 3 CDRs of the heavy chain variable region have the following amino acid sequences:
HCDR1: GYTFTNYG (SEQ ID NO: 58)
HCDR2: INTYTGEP (SEQ ID NO: 59)
HCDR3: AKYPHYYGSSHWYFDV (SEQ ID NO: 60)

The 3 CDRs of the heavy chain variable region have the following amino acid sequences:
LCDR1: QDISNY (SEQ ID NO: 61)
LCDR2: FTS (SEQ ID NO: 62)
LCDR3: QQYSTVPWT (SEQ ID NO: 63)

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of CTLA4 protein (cytotoxic T-lymphocyte-associated antigen 4), it includes the full length of CTLA4 protein, or the extracellular fragment CTLA4 ECD of CTLA4 or a fragment comprising CTLA4 ECD; also included are fusion proteins of CTLA4 ECD, such as a fragment fused to an Fc protein fragment of a mouse or human IgG (mFc or hFc). However, those skilled in the art will appreciate that in the amino acid sequence of the CTLA4 protein, mutations or variations (including but not limited to, substitutions, deletions and/or additions) can be naturally produced or artificially introduced without affecting biological functions thereof. Therefore, in the present invention, the term "CTLA4 protein" shall include all such sequences, including their natural or artificial variants. In addition, when describing a sequence fragment of the CTLA4 protein, it also includes the corresponding sequence fragments in their natural or artificial variants.

As used herein, when referring to the amino acid sequence of PD-1 protein (NCBI GenBank: NM_005018), it includes the full length of PD-1 protein, or the extracellular fragment PD-1ECD of PD-1 or a fragment comprising PD-1ECD; also included are fusion proteins of PD-1ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions and/or additions) can be naturally produced or artificially introduced without affecting biological functions thereof. Therefore, in the present invention, the term "PD-1 protein" should include all such sequences, including their natural or artificial variants. In addition, when describing a sequence fragment of the PD-1 protein, it also includes the corresponding sequence fragments in their natural or artificial variants.

As used herein, unless otherwise specified, the B7 is B7-1 and/or B7-2; specific sequences thereof are those known in the prior art, and reference may be made to sequences disclosed in the existing literature or GenBank. For example, B7-1 (CD80, NCBI Gene ID: 941); B7-2 (CD86, NCBI Gene ID: 942).

As used herein, when referring to the amino acid sequence of VEGFA protein (GenBank ID: NP_001165097.1), it includes the full length of the VEGFA protein, as well as a fusion protein of VEGFA, such as a fragment fused to an Fc protein fragment of mouse or human IgG (mFc or hFc). However, those skilled in the art will appreciate that in the amino acid sequence of the VEGFA protein, mutations or variations (including but not limited to, substitutions, deletions and/or additions) may naturally occur or can be artificially introduced without affecting biological functions thereof. Therefore, in the present invention, the term "VEGFA protein" shall include all such sequences, including their natural or artificial variants. In addition, when describing a sequence fragment of the VEGFA protein, it also includes the corresponding sequence fragments in their natural or artificial variants.

As used herein, when referring to the amino acid sequence of VEGFR2 protein (also known as KDR, GenBank ID: NP_002244), it includes the full length of the VEGFR2 protein, or the extracellular fragment VEGFR2-ECD of VEGFR2, or a fragment comprising VEGFR2-ECD, and it also includes a fusion protein of VEGFR2-ECD, such as a fragment fused to an Fc protein fragment of mouse or human IgG (mFc or hFc). However, those skilled in the art will appreciate that in the amino acid sequence of the VEGFR2 protein, mutations or variations (including but not limited to, substitutions, deletions and/or additions) may naturally occur or can be artificially introduced without affecting biological functions thereof. Therefore, in the present invention, the term "VEGFR2 protein" shall include all such sequences, including their natural or artificial variants. In addition, when describing a sequence fragment of the VEGFR2 protein, it also includes the corresponding sequence fragments in their natural or artificial variants. As used herein, unless otherwise specified, the VEGFR is VEGFR1 and/or VEGFR2; specific sequences thereof are those known in the prior art, and reference may be made to sequences disclosed in the existing literature or GenBank. For example, VEGFR1 (VEGFR1, NCBI Gene ID: 2321); VEGFR2 (VEGFR2, NCBI Gene ID: 3791).

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, 6, γ, α and ε, and the antibodies are defined as IgM, IgD, IgG, IgA and IgE isotypes, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of classical complement system. The V_{H} and V_{L} regions can be further subdivided into hypervariable regions (called complementarity determining regions, or CDRs) and conservative regions called framework regions (FRs) that are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form an antibody binding site. The assignment of amino acids to each region or domain follows the definition of Kabat Sequences of Proteins of Immunological Interest (National Institutes of Health, Bethesda, MD. (1987 and 1991)), Chothia & Lesk, (1987) J. Mol. Biol., 196:901-917, or Chothia et al. (1989) Nature, 342:878-883. In particular, the heavy chain may further comprise more than 3 CDRs, such as 6, 9, or 12. For example, in the bifunctional antibody of the present invention, the heavy chain may be an scFv with the C terminus of the heavy chain of IgG antibody linked to another antibody, and in this case, the heavy chain comprises 9 CDRs. The term "antibody" is not limited by any specific method for producing the antibody. For example, the antibody includes, in particular, a recombinant antibody, a monoclonal antibody and a polyclonal antibody. The antibody can be antibodies of different isotypes, such as IgG (e.g., subtypes IgG1, IgG2, IgG3 or IgG4), IgA1, IgA2, IgD, IgE or IgM.

Antigen-binding fragments (e.g., the above mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies.

As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technique first reported by Kohler et al. (Kohler et al., Nature, 256:495, 1975), but can also be obtained using DNA recombination (see, e.g., U.S. Patent No. 4,816,567).

As used herein, the term "humanized antibody" refers to an antibody or antibody fragment obtained when all or a part of the CDRs of a human immunoglobulin (receptor antibody) is replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522 525 (1986); Reichmann et al., Nature, 332:323 329 (1988); Presta, Curr. Op Struct. Biol., 2:593 596 (1992); and Clark, Immunol. Today 21: 397 402 (2000).

As used herein, the term "epitope" refers to a site on the antigen that an immunoglobulin or antibody specifically binds to. "Epitope" is also referred to in the art as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14 or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "E. coli expression system" refers to an expression system consisting of E. coli (strain) and a vector, wherein the E. coli (strain) is derived from a commercially available strain, such as but not limited to GI698, ER2566, BL21 (DE3), B834 (DE3), and BLR (DE3).

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages, such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *Escherichia coli* or *bacillus subtilis*, fungal cells such as yeast cells or *Aspergillus*, insect cells such as S2 drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) less than about 10⁻⁵ M, such as less than about 10⁻⁶ M, 10⁻⁷ M, 10⁻⁸ M, 10⁻⁹ M or 10⁻¹⁰ M or less. In some embodiments of the present invention, the term "target" refers to specific binding.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less, for example, as measured by a BIACORE surface plasmon resonance (SPR) instrument or a Fortebio Octet system.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable adjuvant" or "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum*, lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount (e.g., for a disease associated with binding of CTLA4 to B7 or CTLA4 overactivity, such as a tumor) is an amount sufficient to prevent, stop, or delay the onset of the disease (e.g., a disease associated with binding of CTLA4 to B7 or CTLA4 overactivity, such as a tumor); a therapeutically effective amount is an amount sufficient to cure or at least partially stop a disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

As used herein, the term "completely eliminated" refers to the absence of binding signal or an extremely weak binding signal as detected by existing instrumentation (e.g., a Fortebio Octet system). In one embodiment of the present invention, the absence of binding signal or the extremely weak binding signal refers to a binding signal (i.e., response) below 0.1 nm.

In the present invention, the terms "first" (e.g., first bispecific antibody, first protein functional region, or first linker fragment) and "second" (e.g., second bispecific antibody, second protein functional region, or second linker fragment) are used for distinguishing or clarity in expression and do not carry typical sequential meanings, unless otherwise specified.

In the present invention, "about" or "approximately" refers to that the value or physical quantity defined fluctuates within a range of 10%, 20% or 30%, unless otherwise specified. For example, about 100 minutes or approximately 100 minutes may be 90 minutes to 110 minutes, 80 minutes to 120 minutes or 70 minutes to 130 minutes.

The earlier applications Chinese Patent Publications CN112300286A and CN112830972A are hereby are incorporated herein by reference in their entirety.

### Beneficial effects of the present invention

The present invention achieves one or more of the following technical effects (1) to (2):
(1) The therapeutic combination of the present invention can effectively prevent or treat a tumor.
(2) The combination of the anti-CTLA4-anti-PD-1 bispecific antibody with the anti-PD-1-anti-VEGFA bispecific antibody have a pharmacological effect of effectively inhibiting tumor growth, which is superior to that of the anti-VEGFA-anti-PD-1 bispecific antibody or the anti-CTLA4-anti-PD-1 bispecific antibody alone, and a synergistic technical effect is achieved.

### BRIEF DESCRIPTION OF THE DRAWINGS

FIG. 1 shows the effect of the anti-CTLA4-anti-PD-1 bispecific antibody in combination with the anti-PD-1-anti-VEGFA bispecific antibody on cytokine IL-2 secretion induced by stimulated PBMC and Raji-PDL1 cell mixed culture.
FIG. 2 shows the detection of the promotion of the anti-CTLA4-anti-PD-1 bispecific antibody in combination with the anti-PD-1-anti-VEGFA bispecific antibody on the secretion of cytokine IFN-γ by mixed lymphocyte reaction.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the product manual. Reagents or instruments used are commercially available conventional products if the manufacturers thereof are not specified.

In the following examples of the present invention:
Human peripheral blood mononuclear cells were isolated and prepared in Akeso Biopharma, Inc., with informed consent of the donor.

Raji-PDL1 is a cell expressing human PD-L1 constructed by Akeso Biopharma, Inc. on the basis of human B cells Raji via transfection.

Ficoll-Paque^{™} PLUS (or Ficoll-Paque PLUS) was purchased from GE Healthcare, Cat. No. 17-1440-02.

Human IL-2 ELISA Kit was purchased from Dakewe Biotech Co., Ltd., Cat. No. 1110202.

RPMI 1640 medium, DMEM medium, Trypsin-EDTA (0.25%) phenol red and Blastidin were all purchased from Gibco.

*Staphylococcus aureus* enterotoxin B (SEB) was purchased from Dianotech, Cat. No. BT202.

FBS was purchased from Excell bio, Cat. No. FSP500.

Mitomycin C (MMC) was purchased from Stressmarq, Cat. No. SIH-246-10MG, Batch No. SM286474.

The sequence of the isotype control, human anti-hen egg lysozyme IgG (anti-HEL antibody, or human IgG, abbreviated as hIgG) is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-146). The hIgG1DM used in the examples is an isotype control antibody with anti-HEL having a hGIDM constant region sequence, prepared in Akeso Biopharma, Inc., Batch No: 20190520.

### Preparation Example 1: Sequence Design of Anti-CTLA4 Antibodies

The amino acid sequences and encoding nucleotide sequences of the heavy and light chains of the anti-CTLA4 antibody 4G10 and its humanized antibodies 4G10H1L1 and 4G10H3L3 are identical to those of 4G10, 4G10H1L1 and 4G10H3L3 in Chinese Patent Publication No. CN106967172A, respectively.
(1) Heavy and light chain variable region sequences of 4G10
   nucleotide sequence of the heavy chain variable region: (372 bp) 
CAGGTCAAGCTGCAGGAGTCTGGACCTGAGCTGGTGAAGCCT
GGAGCTTCAATGAAGATATCCTGCAAGGCTTCTGGTTACTCAT
TCACTGGCTACACCATGAACTGGGTGAAGCAGAGCCATGGAA
AGAACCTTGAATGGATTGGACTTATTAATCCTTACAATAATATT
ACTAACTACAACCAGAAGTTCATGGGCAAGGCCACATTTACTG
TAGACAAGTCATCCAGCACAGCCTACATGGAACTCCTCAGACT
GACATCTGAAGACTCTGGAGTCTATTTCTGTGCAAGACTCGAC
TATAGGTCTTATTGGGGCCAAGGGACTCTGGTCACTGTCTCTG
CAGCCAAAACGACACCCCCATCTGTCTAT (SEQ ID NO: 1)
   its encoded amino acid sequence: (124 aa) 
QVKLQESGPELVKPGASMKISCKASGYSFTGYTMNWVKQSHGK
NLEWIGLINPYNNITNYNQKFMGKATFTVDKSSSTAYMELLRLTS
EDSGVYFCARLDYRSYWGQGTLVTVSAAKTTPPSVY (SEQ ID
NO: 2)
   nucleotide sequence of the light chain variable region: (378 bp) 
CAGGCTGTTGTGACTCAGGAATCTGCACTCACCACATCACCT
GGTGAAACAGTCACACTCACTTGTCGCTCAAGTACTGGGGCT
GTTACAACTAGTAACTTTGCCAACTGGGTCCAAGAAAAACCAG
ATCATTTATTCACTAGTCTAATAGGTGGTACCAACAACCGAGCT
CCAGGTGTTCCTGCCAGATTCTCAGGCTCCCTGATTGGAGAC
AAGGCTGCCCTCACCATCACAGGGGCACAGACTGAGGATGAG
GCAATATATTTCTGTGCTCTATGGTACAGCAACCATTGGGTGTT
CGGTGGAGGAACCAAACTGACTGTCCTAGGCCAGCCCAAGTC
TTCGCCATCAGTCACCCTGTTTCAAGGGCAATTCTGC(SEQ ID
NO: 3)
   its encoded amino acid sequence: (126 aa) 
QAVVTQESALTTSPGETVTLTCRSSTGAVTTSNFANWVQEKPDHL
FTSLIGGTNNRAPGVPARFSGSLIGDKAALTITGAQTEDEAIYFCA
LWYSNHWVFGGGTKLTVLGQPKSSPSVTLFQGQFC(SEQ ID NO:
4)
(2) Heavy and light chain variable region sequences of humanized monoclonal antibody 4G10H1L1
   The nucleotide sequence of the heavy chain variable region (4G10H1V): (345 bp) 
CAGGTGCAGCTGGTGGAGTCTGGGGCCGAGCTGGTGAAGCC
CGGCGCCTCCATGAAGATCTCTTGCAAGGCCAGCGGATACAG
TTTCACTGGCTATACCATGAACTGGGTCAAACAGGCTCCAGGA
CAGGGACTGGAGTGGATCGGGCTGATTAATCCTTACAACAACA
TCACCAACTACAACCAGAAGTTCATGGGAAAAGCAACCTTTAC
AGTGGACAAGAGCATTTCCACAGCCTACATGGAACTGAGCCG
GCTGACTTCAGACGATAGCGGGGTCTATTTTTGTGCAAGGCT
GGATTATCGCTCTTACTGGGGGCAGGGAACTCTGGTCACTGT
CTCCGCT(SEQ ID NO: 5)
   its encoded amino acid sequence: (115 aa) 
QVQLVESGAELVKPGASMKISCKASGYSFTGYTMNWVKQAPGQ
GLEWIGLINPYNNITNYNQKFMGKATFTVDKSISTAYMELSRLTS
DDSGVYFCARLDYRSYWGQGTLVTVSA(SEQ ID NO: 6)
   The nucleotide sequence of the light chain variable region (4G10L1V): (327 bp) 
CAGGCTGTCGTCACTCAGGAACCTTCACTGACTGTGAGCCCA
GGAGGAACTGTCACCCTGACATGCGGAAGCTCCACCGGAGCA
GTGACCACATCCAACTTCGCCAATTGGGTCCAGGAAAAGCCA
GGCCAGGCATTTCGATCCCTGATCGGAGGCACAAACAATCGG
 
GCTTCTTGGGTGCCCGCAAGATTCTCAGGAAGCCTGCTGGGG
GGAAAAGCCGCTCTGACCATTAGTGGCGCTCAGCCTGAGGAC
GAAGCCGAGTACTTCTGCGCTCTGTGGTATAGCAACCACTGG
GTGTTTGGCGGGGGAACAAAGCTGACTGTGCTG(SEQ ID NO:
7)
   its encoded amino acid sequence: (109 aa) 
QAVVTQEPSLTVSPGGTVTLTCGSSTGAVTTSNFANWVQEKPGQ
AFRSLIGGTNNRASWVPARFSGSLLGGKAALTISGAQPEDEAEYF
CALWYSNHWVFGGGTKLTVL(SEQ ID NO: 8)
(3) Heavy and light chain variable region sequences of humanized monoclonal antibody 4G10H3L3
   The nucleotide sequence of the heavy chain variable region (4G10H3V): (345 bp) 
CAGGTGCAGCTGGTCGAGTCTGGGGCCGAAGTGAAGAAACC
CGGCGCCTCAGTGAAGGTCAGCTGCAAGGCCAGCGGGTACA
GTTTCACTGGATATACCATGAACTGGGTCCGACAGGCCCCTGG
CCAGGGGCTGGAGTGGATCGGCCTGATTAACCCTTACAACAA
CATCACTAACTACGCACAGAAGTTCCAGGGGAGAGTGACCTT
TACAGTGGACACCAGCATTTCCACAGCCTACATGGAACTGTCC
CGGCTGAGATCTGACGATACAGGCGTGTACTTCTGCGCTAGG
CTGGATTACCGCAGCTATTGGGGACAGGGCACACTGGTGACT
GTCAGCGCA(SEQ ID NO: 9)
   The encoded amino acid sequence (4G10H3V): (115 aa) 
QVQLVESGAEVKKPGASVKVSCKASGYSFTGYTMNWVRQAPGQ
GLEWIGLINPYNNITNYAQKFQGRVTFTVDTSISTAYMELSRLRS
DDTGVYFCARLDYRSYWGQGTLVTVSA(SEQ ID NO: 10)
   The nucleotide sequence of the light chain variable region (4G10L3V): (327 bp) 
CAGGCTGTCGTCACTCAGGAACCTTCACTGACCGTGTCTCCT
GGCGGGACTGTCACCCTGACATGCGGCAGCTCCACAGGGGC
CGTGACCACAAGTAACTTCCCAAATTGGGTCCAGCAGAAGCC
AGGACAGGCTCCCCGGAGTCTGATCGGAGGCACCAACAACAA
GGCCAGCTGGACACCCGCACGGTTCAGCGGCAGCCTGCTGG
GCGGCAAGGCCGCTCTGACAATTAGCGGAGCCCAGCCTGAGG
ACGAAGCCGAGTACTATTGCGCTCTGTGGTACTCCAACCACTG
GGTGTTCGGCGGCGGCACCAAGCTGACTGTGCTG(SEQ ID NO:
11)
   The encoded amino acid sequence (4G10L3V): (109 aa)

QAVVTQEPSLTVSPGGTVTLTCGSSTGAVTTSNFPNWVQQKPGQ
APRSLIGGTNNKASWTPARFSGSLLGGKAALTISGAQPEDEAEYY
CALWYSNHWVFGGGTKLTVL(SEQ ID NO: 12)

### Preparation Example 2: Sequence Design of Anti-PD-1 Antibody 14C12 and Its Humanized Antibody 14C12H1L1

The amino acid sequences and encoding nucleotide sequences of the heavy and light chains of the anti-PD-1 antibody 14C12 and its humanized antibodies 14C12H1L1 are identical to those of 14C12 and 14C12H1L1 in Chinese Patent Publication No. CN106967172A, respectively.
(1) Heavy and light chain variable region sequences of 14C12
   nucleotide sequence of the heavy chain variable region: (354 bp) 
GAGGTCAAACTGGTGGAGAGCGGCGGCGGGCTGGTGAAGCCC
GGCGGGTCACTGAAACTGAGCTGCGCCGCTTCCGGCTTCGCCT
TTAGCTCCTACGACATGTCATGGGTGAGGCAGACCCCTGAGAA
GCGCCTGGAATGGGTCGCTACTATCAGCGGAGGCGGGCGATAC
ACCTACTATCCTGACTCTGTCAAAGGGAGATTCACAATTAGTCG
 
GGATAACGCCAGAAATACTCTGTATCTGCAGATGTCTAGTCTGC
GGTCCGAGGATACAGCTCTGTACTATTGTGCAAACCGGTACGG
CGAAGCATGGTTTGCCTATTGGGGACAGGGCACCCTGGTGACA
GTCTCTGCC(SEQ ID NO: 13)
   its encoded amino acid sequence: (118 aa) 
EVKLVESGGGLVKPGGSLKLSCAASGFAFSSYDMSWVRQTPEKRL
EWVATISGGGRYTYYPDSVKGRFTISRDNARNTLYLQMSSLRSEDT
ALYYCANRYGEAWFAYWGQGTLVTVSA(SEQ ID NO: 14)
   nucleotide sequence of the light chain variable region: (321 bp) 
GACATTAAGATGACACAGTCCCCTTCCTCAATGTACGCTAGCCT
GGGCGAGCGAGTGACCTTCACATGCAAAGCATCCCAGGACATC
AACACATACCTGTCTTGGTTTCAGCAGAAGCCAGGCAAAAGCC
CCAAGACCCTGATCTACCGGGCCAATAGACTGGTGGACGGGGT
CCCCAGCAGATTCTCCGGATCTGGCAGTGGGCAGGATTACTCC
CTGACCATCAGCTCCCTGGAGTATGAAGACATGGGCATCTACTA
TTGCCTGCAGTATGATGAGTTCCCTCTGACCTTTGGAGCAGGC
ACAAAACTGGAACTGAAG(SEQ ID NO: 15)
   its encoded amino acid sequence: (107 aa) 
DIKMTQSPSSMYASLGERVTFTCKASQDINTYLSWFQQKPGKSPK
TLIYRANRLVDGVPSRFSGSGSGQDYSLTISSLEYEDMGIYYCLQY
DEFPLTFGAGTKLELK(SEQ ID NO: 16)
(2) Heavy and light chain variable region and heavy and light chain sequences of humanized monoclonal antibody 14C12H1L1
   nucleotide sequence of the heavy chain variable region: (354 bp) 
GAAGTGCAGCTGGTCGAGTCTGGGGGAGGGCTGGTGCAGCC
CGGCGGGTCACTGCGACTGAGCTGCGCAGCTTCCGGATTCGC
CTTTAGCTCCTACGACATGTCCTGGGTGCGACAGGCACCAGG
 
AAAGGGACTGGATTGGGTCGCTACTATCTCAGGAGGCGGGAG
ATACACCTACTATCCTGACAGCGTCAAGGGCCGGTTCACAATC
TCTAGAGATAACAGTAAGAACAATCTGTATCTGCAGATGAACA
GCCTGAGGGCTGAGGACACCGCACTGTACTATTGTGCCAACC
GCTACGGGGAAGCATGGTTTGCCTATTGGGGGCAGGGAACCC
TGGTGACAGTCTCTAGT (SEQ ID NO: 17)
   its encoded amino acid sequence: (118 aa) 
EVQLVESGGGLVQPGGSLRLSCAASGFAFSSYDMSWVRQAPGKG
LDWVATISGGGRYTYYPDSVKGRFTISRDNSKNNLYLQMNSLRA
EDTALYYCANRYGEAWFAYWGQGTLVTVSS(SEQ ID NO: 18)
   nucleotide sequence of the light chain variable region: (321 bp) 
GACATTCAGATGACTCAGAGCCCCTCCTCCATGTCCGCCTCTG
TGGGCGACAGGGTCACCTTCACATGCCGCGCTAGTCAGGATA
TCAACACCTACCTGAGCTGGTTTCAGCAGAAGCCAGGGAAAA
GCCCCAAGACACTGATCTACCGGGCTAATAGACTGGTGTCTG
GAGTCCCAAGTCGGTTCAGTGGCTCAGGGAGCGGACAGGACT
ACACTCTGACCATCAGCTCCCTGCAGCCTGAGGACATGGCAA
CCTACTATTGCCTGCAGTATGATGAGTTCCCACTGACCTTTGG
CGCCGGGACAAAACTGGAGCTGAAG(SEQ ID NO: 19)
   its encoded amino acid sequence: (107 aa) 
DIQMTQSPSSMSASVGDRVTFTCRASQDINTYLSWFQQKPGKSP
KTLIYRANRLVSGVPSRFSGSGSGQDYTLTISSLQPEDMATYYCL
QYDEFPLTFGAGTKLELK(SEQ ID NO: 20)
   The DNA sequence of the heavy chain (14C12H1) of 14C12H1L1: (1344 bp) 
GAAGTGCAGCTGGTCGAGTCTGGGGGAGGGCTGGTGCAGCCC
GGCGGGTCACTGCGACTGAGCTGCGCAGCTTCCGGATTCGCCT
 
TTAGCTCCTACGACATGTCCTGGGTGCGACAGGCACCAGGAAA
GGGACTGGATTGGGTCGCTACTATCTCAGGAGGCGGGAGATAC
ACCTACTATCCTGACAGCGTCAAGGGCCGGTTCACAATCTCTAG
AGATAACAGTAAGAACAATCTGTATCTGCAGATGAACAGCCTGA
GGGCTGAGGACACCGCACTGTACTATTGTGCCAACCGCTACGG
GGAAGCATGGTTTGCCTATTGGGGGCAGGGAACCCTGGTGACA
GTCTCTAGTGCCAGCACCAAAGGACCTAGCGTGTTTCCTCTCG
CCCCCTCCTCCAAAAGCACCAGCGGAGGAACCGCTGCTCTCGG
ATGTCTGGTGAAGGACTACTTCCCTGAACCCGTCACCGTGAGC
TGGAATAGCGGCGCTCTGACAAGCGGAGTCCATACATTCCCTG
CTGTGCTGCAAAGCAGCGGACTCTATTCCCTGTCCAGCGTCGT
CACAGTGCCCAGCAGCAGCCTGGGCACCCAGACCTACATCTGT
AACGTCAACCACAAGCCCTCCAACACCAAGGTGGACAAGAAAG
TGGAGCCCAAATCCTGCGACAAGACACACACCTGTCCCCCCTG
TCCTGCTCCCGAACTCCTCGGAGGCCCTAGCGTCTTCCTCTTT
CCTCCCAAACCCAAGGACACCCTCATGATCAGCAGAACCCCTG
AAGTCACCTGTGTCGTCGTGGATGTCAGCCATGAGGACCCCGA
GGTGAAATTCAACTGGTATGTCGATGGCGTCGAGGTGCACAAC
GCCAAAACCAAGCCCAGGGAGGAACAGTACAACTCCACCTACA
GGGTGGTGTCCGTGCTGACAGTCCTCCACCAGGACTGGCTGA
ACGGCAAGGAGTACAAGTGCAAGGTGTCCAACAAGGCTCTCCC
TGCCCCCATTGAGAAGACCATCAGCAAGGCCAAAGGCCAACCC
AGGGAGCCCCAGGTCTATACACTGCCTCCCTCCAGGGACGAAC
TCACCAAGAACCAGGTGTCCCTGACCTGCCTGGTCAAGGGCTT
TTATCCCAGCGACATCGCCGTCGAGTGGGAGTCCAACGGACAG
CCCGAGAATAACTACAAGACCACCCCTCCTGTCCTCGACTCCG
ACGGCTCCTTCTTCCTGTACAGCAAGCTGACCGTGGACAAAAG
CAGGTGGCAGCAGGGAAACGTGTTCTCCTGCAGCGTGATGCA
CGAAGCCCTCCACAACCACTACACCCAGAAAAGCCTGTCCCTG
 AGCCCCGGCAAA(SEQ ID NO: 21)
   its encoded amino acid sequence: (448 aa) 
EVQLVESGGGLVQPGGSLRLSCAASGFAFSSYDMSWVRQAPGKG
LDWVATISGGGRYTYYPDSVKGRFTISRDNSKNNLYLQMNSLRAE
DTALYYCANRYGEAWFAYWGQGTLVTVSSASTKGPSVFPLAPSSK
STSGGTAALGCLVKDYFPEPVTVSWNSGALTSGVHTFPAVLQSSGL
YSLSSVVTVPSSSLGTQTYICNVNHKPSNTKVDKKVEPKSCDKTHT
CPPCPAPELLGGPSVFLFPPKPKDTLMISRTPEVTCVVVDVSHEDP
EVKFNWYVDGVEVHNAKTKPREEQYNSTYRVVSVLTVLHQDWL
NGKEYKCKVSNKALPAPIEKTISKAKGQPREPQVYTLPPSRDELTK
NQVSLTCLVKGFYPSDIAVEWESNGQPENNYKTTPPVLDSDGSFFL
YSKLTVDKSRWQQGNVFSCSVMHEALHNHYTQKSLSLSPGK(SEQ
ID NO: 22)
   The DNA sequence of the light chain (14C12L1) of 14C12H1L1: (642 bp) 
GACATTCAGATGACTCAGAGCCCCTCCTCCATGTCCGCCTCTG
TGGGCGACAGGGTCACCTTCACATGCCGCGCTAGTCAGGATAT
CAACACCTACCTGAGCTGGTTTCAGCAGAAGCCAGGGAAAAGC
CCCAAGACACTGATCTACCGGGCTAATAGACTGGTGTCTGGAG
TCCCAAGTCGGTTCAGTGGCTCAGGGAGCGGACAGGACTACA
CTCTGACCATCAGCTCCCTGCAGCCTGAGGACATGGCAACCTA
CTATTGCCTGCAGTATGATGAGTTCCCACTGACCTTTGGCGCCG
GGACAAAACTGGAGCTGAAGCGAACTGTGGCCGCTCCCTCCG
TCTTCATTTTTCCCCCTTCTGACGAACAGCTGAAATCAGGCACA
GCCAGCGTGGTCTGTCTGCTGAACAATTTCTACCCTAGAGAGG
CAAAAGTGCAGTGGAAGGTCGATAACGCCCTGCAGTCCGGCAA
CAGCCAGGAGAGTGTGACTGAACAGGACTCAAAAGATAGCACC
TATTCCCTGTCTAGTACACTGACTCTGTCCAAGGCTGATTACGA
GAAGCACAAAGTGTATGCATGCGAAGTGACACATCAGGGACTG
 
TCAAGCCCCGTGACTAAGTCTTTTAACCGGGGCGAATGT(SEQ
ID NO: 23)
   its encoded amino acid sequence: (214 aa)

DIQMTQSPSSMSASVGDRVTFTCRASQDINTYLSWFQQKPGKSPK
TLIYRANRLVSGVPSRFSGSGSGQDYTLTISSLQPEDMATYYCLQY
DEFPLTFGAGTKLELKRTVAAPSVFIFPPSDEQLKSGTASVVCLLN
NFYPREAKVQWKVDNALQSGNSQESVTEQDSKDSTYSLSSTLTLS
KADYEKHKVYACEVTHQGLSSPVTKSFNRGEC(SEQ ID NO: 24)

### Preparation Example 3: Sequence Design of Bifunctional Antibodies BiAb001(M), BiAb002(M), BiAb003(M) and BiAb004(M)

The structural patterns of the bifunctional antibodies BiAb001(M), BiAb002(M), BiAb003(M) and BiAb004(M) are in the Morrison format (IgG-scFv), i.e., C termini of two heavy chains of one IgG antibody are separately linked to the scFv fragment of another antibody via linker fragments. The components for the heavy and light chain design are shown in Table A below.

**Table A: Sequence design of BiAb001(M), BiAb002(M), BiAb003(M) and BiAb004(M)**

| Bifunctional antibody | Immunoglobulin moiety | | Linker fragment | scFv moiety |
|---|---|---|---|---|
| | Heavy chain | Light chain | | |
| BiAb001(M) | 14C12H1 | 14C12L1 | Linker1 | 4G10H1V(M)-Linker2 -4G10L1V(M) |
| BiAb002(M) | 14C12H1 | 14C12L1 | Linker2 | 4G10H1V(M)-Linker2 -4G10L1V(M) |
| BiAb003(M) | 14C12H1 | 14C12L1 | Linker1 | 4G10H3V(M)-Linker2 -4G10L3V(M) |
| BiAb004(M) | 14C12H1 | 14C12L1 | Linker2 | 4G10H3V(M)-Linker2 -4G10L3V(M) |

In Table A above:
The amino acid sequence of Linker1 is (GGGGS)3 (SEQ ID NO: 25).
The amino acid sequence of Linker2 is (GGGGS)4 (SEQ ID NO: 26).

In Table A above, scFv fragments 4G10H1V(M), 4G10L1V(M), 4G10H3V(M) and 4G10L3V(M) of BiAb001(M), BiAb002(M), BiAb003(M) and BiAb004(M) antibodies comprised mutations in individual amino acids of framework regions on the basis of 4G10H1V, 4G10L1V, 4G10H3V and 4G10L3V, respectively, which effectively optimized the structure of the antibodies and improved their efficacy.
(1) 4G10H1V(M): (115 aa, mutation positions underlined in the amino acid sequence based on 4G10H1V) 
QVQLVESGAELVKPGASMKISCKASGYSFTGYTMNWVKQAPGQ
CLEWIGLINPYNNITNYNQKFMGKATFTVDKSISTAYMELSRLTS
DDSGVYFCARLDYRSYWGQGTLVTVSA(SEQ ID NO: 41)
(2) 4G10L1V(M): (110 aa, mutation positions underlined in the amino acid sequence based on 4G10L1V) 
QAVVTQEPSLTVSPGGTVTLTCGSSTGAVTTSNFANWVQEKPGQ
AFRSLIGGTNNRASWVPARFSGSLLGGKAALTISGAQPEDEAEYF
CALWYSNHWVFGCGTKLTVLR(SEQ ID NO: 42)
(3) 4G10H3V(M): (115 aa, mutation positions underlined in the amino acid sequence based on 4G10H3V) 
QVQLVESGAEVKKPGASVKVSCKASGYSFTGYTMNWVRQAPGQ
CLEWIGLINPYNNITNYAQKFQGRVTFTVDTSISTAYMELSRLRSD
DTGVYFCARLDYRSYWGQGTLVTVSA(SEQ ID NO: 43)
(4) 4G10L3V(M): (110 aa, mutation positions underlined in the amino acid sequence based on 4G10L3V)

QAVVTQEPSLTVSPGGTVTLTCGSSTGAVTTSNFPNWVQQKPGQ
APRSLIGGTNNKASWTPARFSGSLLGGKAALTISGAQPEDEAEYY
CALWYSNHWVFGCGTKLTVLR(SEQ ID NO: 44)

For distinguishment from the mutated antibodies hereinafter, BiAb004(M) is also referred to as BiAb004(hG1WT) in the examples of the present invention. BiAb004(M) described above is the "wild-type", comprising an Ig gamma-1 chain C region (ACCESSION: P01857) as the heavy chain constant region and an Ig kappa chain C region (ACCESSION: P01834) as the light chain constant region.

### Preparation Example 4: Non-Variable Region Amino Acid Mutation Design Based on Humanized Bifunctional Antibody BiAb004

On the basis of BiAb004(hG1WT) obtained in Preparation Example 3, BiAb004(hG 1 TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain.
DNA sequence of heavy chain of the immunoglobulin moiety in BiAb004(hG1TM): (1344 bp, mutation positions underlined) 
GAAGTGCAGCTGGTCGAGTCTGGGGGAGGGCTGGTGCAGCC
CGGCGGGTCACTGCGACTGAGCTGCGCAGCTTCCGGATTCGC
CTTTAGCTCCTACGACATGTCCTGGGTGCGACAGGCACCAGG
AAAGGGACTGGATTGGGTCGCTACTATCTCAGGAGGCGGGAG
ATACACCTACTATCCTGACAGCGTCAAGGGCCGGTTCACAATC
TCTAGAGATAACAGTAAGAACAATCTGTATCTGCAGATGAACA
GCCTGAGGGCTGAGGACACCGCACTGTACTATTGTGCCAACC
GCTACGGGGAAGCATGGTTTGCCTATTGGGGGCAGGGAACCC
 
TGGTGACAGTCTCTAGTGCCAGCACCAAAGGGCCCAGCGTGT
TTCCTCTCGCCCCCTCCTCCAAAAGCACCAGCGGAGGAACCG
CTGCTCTCGGATGTCTGGTGAAGGACTACTTCCCTGAACCCG
TCACCGTGAGCTGGAATAGCGGCGCTCTGACAAGCGGAGTCC
ATACATTCCCTGCTGTGCTGCAAAGCAGCGGACTCTATTCCCT
GTCCAGCGTCGTCACAGTGCCCAGCAGCAGCCTGGGCACCCA
GACCTACATCTGTAACGTCAACCACAAGCCCTCCAACACCAAG
GTGGACAAGAAAGTGGAGCCCAAATCCTGCGACAAGACACAC
ACCTGTCCCCCCTGTCCTGCTCCCGAAGCTGCTGGAGCCCCT
AGCGTCTTCCTCTTTCCTCCCAAACCCAAGGACACCCTCATGA
TCAGCAGAACCCCTGAAGTCACCTGTGTCGTCGTGGATGTCA
GCCATGAGGACCCCGAGGTGAAATTCAACTGGTATGTCGATG
GCGTCGAGGTGCACAACGCCAAAACCAAGCCCAGGGAGGAA
CAGTACAACTCCACCTACAGGGTGGTGTCCGTGCTGACAGTC
CTCCACCAGGACTGGCTGAACGGCAAGGAGTACAAGTGCAAG
GTGTCCAACAAGGCTCTCCCTGCCCCCATTGAGAAGACCATC
AGCAAGGCCAAAGGCCAACCCAGGGAGCCCCAGGTCTATACA
CTGCCTCCCTCCAGGGACGAACTCACCAAGAACCAGGTGTCC
CTGACCTGCCTGGTCAAGGGCTTTTATCCCAGCGACATCGCC
GTCGAGTGGGAGTCCAACGGACAGCCCGAGAATAACTACAAG
ACCACCCCTCCTGTCCTCGACTCCGACGGCTCCTTCTTCCTGT
ACAGCAAGCTGACCGTGGACAAAAGCAGGTGGCAGCAGGGA
AACGTGTTCTCCTGCAGCGTGATGCACGAAGCCCTCCACAAC
CACTACACCCAGAAAAGCCTGTCCCTGAGCCCCGGCAAA(SEQ
ID NO: 39)
Amino acid sequence of heavy chain of the immunoglobulin moiety in BiAb004(hG1TM): (448 aa, mutation positions underlined) EVQLVESGGGLVQPGGSLRLSCAASGFAFSSYDMSWVRQAPGKG 
LDWVATISGGGRYTYYPDSVKGRFTISRDNSKNNLYLQMNSLRAE
DTALYYCANRYGEAWFAYWGQGTLVTVSSASTKGPSVFPLAPSSK
STSGGTAALGCLVKDYFPEPVTVSWNSGALTSGVHTFPAVLQSSGL
YSLSSVVTVPSSSLGTQTYICNVNHKPSNTKVDKKVEPKSCDKTHT
CPPCPAPEAAGAPSVFLFPPKPKDTLMISRTPEVTCVVVDVSHEDP
EVKFNWYVDGVEVHNAKTKPREEQYNSTYRVVSVLTVLHQDWL
NGKEYKCKVSNKALPAPIEKTISKAKGQPREPQVYTLPPSRDELTK
NQVSLTCLVKGFYPSDIAVEWESNGQPENNYKTTPPVLDSDGSFFL
YSKLTVDKSRWQQGNVFSCSVMHEALHNHYTQKSLSLSPGK(SEQ
ID NO: 40)
BiAb004(hG1TM) and BiAb004(hG1WT) share the same DNA sequence of light chain and the same encoded amino acid sequence. The specific sequence is shown in Preparation Example 3.

### Preparation Example 5: Preparation of Anti-VEGFA Antibody Bevacizumab

For the amino acid sequences of the heavy chain variable region and the light chain variable region of the commercially available anti-VEGFA monoclonal antibody Avastin (bevacizumab), refer to the Chinese Patent Publication No. CN1259962A. Genscript was entrusted to synthesize the nucleotide sequences encoding the heavy chain variable region and the light chain variable region.
Amino acid sequence of bevacizumab heavy chain variable region (Bevacizumab-Hv): (123 aa) 


(SEQ ID NO:
45)
Nucleotide sequence encoding bevacizumab heavy chain variable region: (369 bp) 
GAGGTGCAGCTGGTCGAGTCCGGGGGGGGGCTGGTGCAGCCA
GGCGGGTCTCTGAGGCTGAGTTGCGCCGCTTCAGGGTACACCT
TCACAAACTATGGAATGAATTGGGTGCGCCAGGCACCAGGAAA
GGGACTGGAGTGGGTCGGCTGGATCAACACTTACACCGGGGA
ACCTACCTATGCAGCCGACTTTAAGCGGCGGTTCACCTTCAGC
CTGGATACAAGCAAATCCACTGCCTACCTGCAGATGAACAGCC
TGCGAGCTGAGGACACCGCAGTCTACTATTGTGCTAAATATCCC
CACTACTATGGGAGCAGCCATTGGTATTTTGACGTGTGGGGGC
AGGGGACTCTGGTGACAGTGAGCAGC(SEQ ID NO: 46)
Amino acid sequence of bevacizumab light chain variable region (Bevacizumab-Lv): (107 aa) 


(SEQ ID NO: 47)
Nucleotide sequence encoding bevacizumab light chain variable region: (321 bp)

GATATTCAGATGACTCAGAGCCCCTCCTCCCTGTCCGCCTCTGT
GGGCGACAGGGTCACCATCACATGCAGTGCTTCACAGGATATT
TCCAACTACCTGAATTGGTATCAGCAGAAGCCAGGAAAAGCAC
CCAAGGTGCTGATCTACTTCACTAGCTCCCTGCACTCAGGAGT
GCCAAGCCGGTTCAGCGGATCCGGATCTGGAACCGACTTTACT
CTGACCATTTCTAGTCTGCAGCCTGAGGATTTCGCTACATACTA
TTGCCAGCAGTATTCTACCGTGCCATGGACATTTGGCCAGGGG
ACTAAAGTCGAGATCAAG(SEQ ID NO: 48)

The heavy chain constant regions were all Ig gamma-1 chain C region, ACCESSION: P01857; the light chain constant regions were all Ig kappa chain C region, ACCESSION: P01834.

The heavy chain cDNA and the light chain cDNA of bevacizumab were each cloned into vector pcDNA3.1, and the recombinant expression plasmid of the antibody bevacizumab was obtained. The recombinant plasmid was used to transfect 293F cells. The 293F cell culture was purified and then detected.

The anti-VEGFA monoclonal antibody Avastin (bevacizumab) was thus obtained.

### Preparation Example 6: Sequence Design of Mutant 14C12H1L1(M) of Anti-PD-1 Humanized Antibody 14C12H1L1

14C12H1L1(M) was obtained by a mutation on an amino acid in the framework region (light chain) of 14C12H1L1.

### Heavy chain variable region 14C12H1(M) of 14C12H1L1(M):

The sequence is identical to the heavy chain variable region 14C12H1 of 14C12H1L1. That is, the amino acid sequence is set forth in SEQ ID NO: 18.
Light chain variable region 14C12L1(M) of 14C12H1L1(M): (108 aa; the mutant position on the basis of 14C12H1L1 underlined in the amino acid sequence)

DIQMTQSPSSMSASVGDRVTFTCRASQDINTYLSWFQQKPGKSPK
TLIYRANRLVSGVPSRFSGSGSGQDYTLTISSLQPEDMATYYCLQY
DEFPLTFGAGTKLELKR(SEQ ID NO: 49)

### Preparation Example 7: Sequence Design of Bispecific Antibodies

### 1. Sequence design

The structure of the bispecific antibody described herein is in the Morrison form (IgG-scFv), i.e., C-termini of two heavy chains of an IgG antibody are each linked to a scFv fragment of another antibody, and the main composition design of the heavy and light chains is as shown in Table B below.

On the basis of the bevacizumab described above, the VP101 antibody comprises the amino acid sequences of the heavy chain variable region and the light chain variable region of the 14C12H1L1(M) as the scFv fragments, and is referred to as VP101(M). Compared with 14C12H1L1, 14C12H1L1(M) demonstrated effectively optimized bispecific antibody structure and improved efficacy.

**Table B: Composition design of heavy and light chains of VP101(M) and VP101(G4M)**

| Bispecific antibody No. | Immunoglobulin moiety | | Linker fragment | scFv moiety |
|---|---|---|---|---|
| | Heavy chain | Light chain | | |
| VP101(M) | Bevacizumab-H | Bevacizumab-L | Linker2 | 14C12H1(M)ᵥ-Linker2-14C12L1(M)_{V} |
| VP101(G4M) | Bevacizumab-G4H | Bevacizumab-L | Linker2 | 14C12H1(M)_{V}-Linker2-14C12L1(M)_{V} |

In Table B above:
(1) Those with "V" label at lower right corner refer to the variable region of the corresponding heavy chain or the variable region of the corresponding light chain. For those without "V" label, the corresponding heavy or light chain is the full length comprising the constant region. The corresponding sequences described in the above preparation examples are referred to for the amino acid sequences of these variable regions or the full lengths and the nucleotide sequences encoding them.
(2) The amino acid sequence of Linker2 is GGGGSGGGGSGGGGSG GGGS(SEQ ID NO: 26) Optionally, the amino acid sequence GGGGSGGGGSGGGGS (SEQ ID NO: 25) may be used as Linker1, replacing the aforementioned Linker 2.
(3) Bevacizumab-H comprises Ig gamma-1 chain C region (ACCESSION: P01857) as the heavy chain constant region.
(4) Bevacizumab-G4H comprises Ig gamma-4 chain C region (ACCESSION: P01861.1) as the heavy chain constant region.

### 2. Expression and purification of antibody VP101(M)

The heavy chain cDNA sequence and the light chain cDNA sequence of VP101(M) were each cloned into vector pUC57simple (provided by Genscript) to obtain plasmids pUC57simple-VP101H and pUC57simple-VP101L, respectively.

pUC57simple-VP101H and pUC57simple-VP101L plasmids were each digested (HindIII&EcoRI), and the heavy and light chains isolated by electrophoresis were separately subcloned into pcDNA3.1 vectors, and recombinant plasmids were extracted to co-transfect 293F cells. After 7 days of cell culture, the culture medium was centrifuged at high speed, and the supernatant was concentrated and loaded onto a HiTrap MabSelect SuRe column. The protein was further subjected to a one-step elution with an elution buffer, and the target sample antibody VP101 was isolated and transferred into PBS by buffer exchange.

### 3. Detection of antibody VP101(M)

The purified sample was added to both a reduced protein electrophoresis loading buffer and a non-reduced protein electrophoresis loading buffer, boiled and subjected to SDS-PAGE electrophoresis analysis.

For differentiation from the mutant antibody in Preparation Example 8, VP101(M) is also referred to as VP101(hG1WT) in the present invention. VP101(M) described above is the "wild-type", comprising an Ig gamma-1 chain C region (ACCESSION: P01857) as the heavy chain constant region and an Ig kappa chain C region (ACCESSION: P01834) as the light chain constant region.
Amino acid sequence of the heavy chain of the immunoglobulin moiety in VP101(hG1WT): (453 aa) 
EVQLVESGGGLVQPGGSLRLSCAASGYTFTNYGMNWVRQAPGK
GLEWVGWINTYTGEPTYAADFKRRFTFSLDTSKSTAYLQMNSLRA
EDTAVYYCAKYPHYYGSSHWYFDVWGQGTLVTVSSASTKGPSVF
PLAPSSKSTSGGTAALGCLVKDYFPEPVTVSWNSGALTSGVHTFPA
VLQSSGLYSLSSVVTVPSSSLGTQTYICNVNHKPSNTKVDKKVEPK
SCDKTHTCPPCPAPELLGGPSVFLFPPKPKDTLMISRTPEVTCVVV
DVSHEDPEVKFNWYVDGVEVHNAKTKPREEQYNSTYRVVSVLTV
LHQDWLNGKEYKCKVSNKALPAPIEKTISKAKGQPREPQVYTLPP
SRDELTKNQVSLTCLVKGFYPSDIAVEWESNGQPENNYKTTPPVLD
SDGSFFLYSKLTVDKSRWQQGNVFSCSVMHEALHNHYTQKSLSLS
PGK(SEQ ID NO: 50)
Nucleotide sequence encoding the heavy chain of the immunoglobulin moiety in VP101(hG1WT): (1359 bp)

GAGGTGCAGCTGGTCGAGTCCGGGGGGGGGCTGGTGCAGCCA
GGCGGGTCTCTGAGGCTGAGTTGCGCCGCTTCAGGGTACACCT
TCACAAACTATGGAATGAATTGGGTGCGCCAGGCACCAGGAAA
GGGACTGGAGTGGGTCGGCTGGATCAACACTTACACCGGGGA
ACCTACCTATGCAGCCGACTTTAAGCGGCGGTTCACCTTCAGC
CTGGATACAAGCAAATCCACTGCCTACCTGCAGATGAACAGCC
TGCGAGCTGAGGACACCGCAGTCTACTATTGTGCTAAATATCCC
CACTACTATGGGAGCAGCCATTGGTATTTTGACGTGTGGGGGC
AGGGGACTCTGGTGACAGTGAGCAGCGCAAGCACCAAAGGGC
CCAGCGTGTTTCCTCTCGCCCCCTCCTCCAAAAGCACCAGCGG
AGGAACCGCTGCTCTCGGATGTCTGGTGAAGGACTACTTCCCT

GAACCCGTCACCGTGAGCTGGAATAGCGGCGCTCTGACAAGCG
GAGTCCATACATTCCCTGCTGTGCTGCAAAGCAGCGGACTCTAT
TCCCTGTCCAGCGTCGTCACAGTGCCCAGCAGCAGCCTGGGCA
CCCAGACCTACATCTGTAACGTCAACCACAAGCCCTCCAACAC
CAAGGTGGACAAGAAAGTGGAGCCCAAATCCTGCGACAAGAC
ACACACCTGTCCCCCCTGTCCTGCTCCCGAACTCCTCGGAGGC
CCTAGCGTCTTCCTCTTTCCTCCCAAACCCAAGGACACCCTCAT
GATCAGCAGAACCCCTGAAGTCACCTGTGTCGTCGTGGATGTC
AGCCATGAGGACCCCGAGGTGAAATTCAACTGGTATGTCGATG
GCGTCGAGGTGCACAACGCCAAAACCAAGCCCAGGGAGGAAC
AGTACAACTCCACCTACAGGGTGGTGTCCGTGCTGACAGTCCT
CCACCAGGACTGGCTGAACGGCAAGGAGTACAAGTGCAAGGT
GTCCAACAAGGCTCTCCCTGCCCCCATTGAGAAGACCATCAGC
AAGGCCAAAGGCCAACCCAGGGAGCCCCAGGTCTATACACTGC
CTCCCTCCAGGGACGAACTCACCAAGAACCAGGTGTCCCTGAC
CTGCCTGGTCAAGGGCTTTTATCCCAGCGACATCGCCGTCGAG
TGGGAGTCCAACGGACAGCCCGAGAATAACTACAAGACCACCC
CTCCTGTCCTCGACTCCGACGGCTCCTTCTTCCTGTACAGCAA
ACTGACCGTCGATAAATCTAGGTGGCAGCAGGGCAACGTGTTC
TCTTGTTCCGTGATGCATGAAGCACTGCACAACCATTATACCCA
GAAGTCTCTGAGCCTGTCCCCCGGCAAG(SEQ ID NO: 51)

For differentiation from the mutant antibody in Preparation Example 8, VP101(G4M) is also referred to as VP101(hG4WT) in the present invention. VP101(G4M) described above is the "wild-type", comprising an Ig gamma-4 chain C region (ACCESSION: P01861.1) as the heavy chain constant region and an Ig kappa chain C region (ACCESSION: P01834) as the light chain constant region.
Amino acid sequence of the heavy chain of the immunoglobulin moiety in VP101(hG4WT): (450 aa) 
EVQLVESGGGLVQPGGSLRLSCAASGYTFTNYGMNWVRQAPGK
GLEWVGWINTYTGEPTYAADFKRRFTFSLDTSKSTAYLQMNSLRA
EDTAVYYCAKYPHYYGSSHWYFDVWGQGTLVTVSSASTKGPSVF
PLAPCSRSTSESTAALGCLVKDYFPEPVTVSWNSGALTSGVHTFPA
VLQSSGLYSLSSVVTVPSSSLGTKTYTCNVDHKPSNTKVDKRVESK
YGPPCPPCPAPEFLGGPSVFLFPPKPKDTLMISRTPEVTCVVVDVS
QEDPEVQFNWYVDGVEVHNAKTKPREEQFNSTYRVVSVLTVLHQ
DWLNGKEYKCKVSNKGLPSSIEKTISKAKGQPREPQVYTLPPSQE
EMTKNQVSLTCLVKGFYPSDIAVEWESNGQPENNYKTTPPVLDSD
GSFFLYSRLTVDKSRWQEGNVFSCSVMHEALHNHYTQKSLSLSLG
K(SEQ ID NO: 52)
The nucleotide sequence encoding the heavy chain of the immunoglobulin moiety in VP101(hG4WT): (1350 bp)

GAGGTGCAGCTGGTCGAGTCCGGGGGGGGGCTGGTGCAGCCA
GGCGGGTCTCTGAGGCTGAGTTGCGCCGCTTCAGGGTACACCT
TCACAAACTATGGAATGAATTGGGTGCGCCAGGCACCAGGAAA
GGGACTGGAGTGGGTCGGCTGGATCAACACTTACACCGGGGA
ACCTACCTATGCAGCCGACTTTAAGCGGCGGTTCACCTTCAGC
CTGGATACAAGCAAATCCACTGCCTACCTGCAGATGAACAGCC
TGCGAGCTGAGGACACCGCAGTCTACTATTGTGCTAAATATCCC
CACTACTATGGGAGCAGCCATTGGTATTTTGACGTGTGGGGGC
AGGGGACTCTGGTGACAGTGAGCAGCGCAAGCACCAAAGGGC
CCTCGGTCTTCCCCCTGGCGCCCTGCTCCAGGAGCACCTCCGA
GAGCACAGCCGCCCTGGGCTGCCTGGTCAAGGACTACTTCCCC
GAACCGGTGACGGTGTCGTGGAACTCAGGCGCCCTGACCAGC
GGCGTGCACACCTTCCCGGCTGTCCTACAGTCCTCAGGACTCT
ACTCCCTCAGCAGCGTGGTGACCGTGCCCTCCAGCAGCTTGGG
CACGAAGACCTACACCTGCAACGTAGATCACAAGCCCAGCAAC

ACCAAGGTGGACAAGAGAGTTGAGTCCAAATATGGTCCCCCAT
GCCCACCATGCCCAGCACCTGAGTTCCTGGGGGGACCATCAGT
CTTCCTGTTCCCCCCAAAACCCAAGGACACTCTCATGATCTCCC
GGACCCCTGAGGTCACGTGCGTGGTGGTGGACGTGAGCCAGG
AAGACCCCGAGGTCCAGTTCAACTGGTACGTGGATGGCGTGGA
GGTGCATAATGCCAAGACAAAGCCGCGGGAGGAGCAGTTCAAC
AGCACGTACCGTGTGGTCAGCGTCCTCACCGTCCTGCACCAGG
ACTGGCTGAACGGCAAGGAGTACAAGTGCAAGGTCTCCAACAA
AGGCCTCCCGTCCTCCATCGAGAAAACCATCTCCAAAGCCAAA
GGGCAGCCCCGAGAGCCACAGGTGTACACCCTGCCCCCATCCC
AGGAGGAGATGACCAAGAACCAGGTCAGCCTGACCTGCCTGG
TCAAAGGCTTCTACCCCAGCGACATCGCCGTGGAGTGGGAGAG
CAATGGGCAGCCGGAGAACAACTACAAGACCACGCCTCCCGTG
CTGGACTCCGACGGCTCCTTCTTCCTCTACAGCAGGCTAACCG
TGGACAAGAGCAGGTGGCAGGAGGGGAATGTCTTCTCATGCTC
CGTGATGCATGAGGCTCTGCACAACCACTACACACAGAAGTCT
CTGAGCCTGTCCCTCGGCAAG(SEQ ID NO: 53)

### Preparation Example 8: Non-Variable Region Amino Acid Mutation Design Based on Humanized Bispecific Antibody VP101(HG1WT)

On the basis of VP101(hG1WT) obtained in Preparation Example 7, VP101(hG1DM) was obtained by introducing a leucine-to-alanine point mutation at position 234 (L234A) and a leucine-to-alanine point mutation at position 235 (L235A) in the heavy chain.
Amino acid sequence of the heavy chain of the immunoglobulin moiety in VP101(hG1DM): (453 aa, mutation positions underlined) 
EVQLVESGGGLVQPGGSLRLSCAASGYTFTNYGMNWVRQAPGK
GLEWVGWINTYTGEPTYAADFKRRFTFSLDTSKSTAYLQMNSLRA
EDTAVYYCAKYPHYYGSSHWYFDVWGQGTLVTVSSASTKGPSVF
 
PLAPSSKSTSGGTAALGCLVKDYFPEPVTVSWNSGALTSGVHTFPA
VLQSSGLYSLSSVVTVPSSSLGTQTYICNVNHKPSNTKVDKKVEPK
SCDKTHTCPPCPAPEAAGGPSVFLFPPKPKDTLMISRTPEVTCVVV
DVSHEDPEVKFNWYVDGVEVHNAKTKPREEQYNSTYRVVSVLTV
LHQDWLNGKEYKCKVSNKALPAPIEKTISKAKGQPREPQVYTLPP
SRDELTKNQVSLTCLVKGFYPSDIAVEWESNGQPENNYKTTPPVLD
SDGSFFLYSKLTVDKSRWQQGNVFSCSVMHEALHNHYTQKSLSLS
PGK(SEQ ID NO: 54)
Nucleotide sequence encoding the heavy chain of the immunoglobulin moiety in VP101(hG1DM): (1359 bp, mutation positions underlined) 
GAGGTGCAGCTGGTCGAGTCCGGGGGGGGGCTGGTGCAGCCA
GGCGGGTCTCTGAGGCTGAGTTGCGCCGCTTCAGGGTACACCT
TCACAAACTATGGAATGAATTGGGTGCGCCAGGCACCAGGAAA
GGGACTGGAGTGGGTCGGCTGGATCAACACTTACACCGGGGA
ACCTACCTATGCAGCCGACTTTAAGCGGCGGTTCACCTTCAGC
CTGGATACAAGCAAATCCACTGCCTACCTGCAGATGAACAGCC
TGCGAGCTGAGGACACCGCAGTCTACTATTGTGCTAAATATCCC
CACTACTATGGGAGCAGCCATTGGTATTTTGACGTGTGGGGGC
AGGGGACTCTGGTGACAGTGAGCAGCGCAAGCACCAAAGGGC
CCAGCGTGTTTCCTCTCGCCCCCTCCTCCAAAAGCACCAGCGG
AGGAACCGCTGCTCTCGGATGTCTGGTGAAGGACTACTTCCCT
GAACCCGTCACCGTGAGCTGGAATAGCGGCGCTCTGACAAGCG
GAGTCCATACATTCCCTGCTGTGCTGCAAAGCAGCGGACTCTAT
TCCCTGTCCAGCGTCGTCACAGTGCCCAGCAGCAGCCTGGGCA
CCCAGACCTACATCTGTAACGTCAACCACAAGCCCTCCAACAC
CAAGGTGGACAAGAAAGTGGAGCCCAAATCCTGCGACAAGAC
ACACACCTGTCCCCCCTGTCCTGCTCCCGAAGCTGCTGGAGGC
CCTAGCGTCTTCCTCTTTCCTCCCAAACCCAAGGACACCCTCAT
GATCAGCAGAACCCCTGAAGTCACCTGTGTCGTCGTGGATGTC
 
AGCCATGAGGACCCCGAGGTGAAATTCAACTGGTATGTCGATG
GCGTCGAGGTGCACAACGCCAAAACCAAGCCCAGGGAGGAAC
AGTACAACTCCACCTACAGGGTGGTGTCCGTGCTGACAGTCCT
CCACCAGGACTGGCTGAACGGCAAGGAGTACAAGTGCAAGGT
GTCCAACAAGGCTCTCCCTGCCCCCATTGAGAAGACCATCAGC
AAGGCCAAAGGCCAACCCAGGGAGCCCCAGGTCTATACACTGC
CTCCCTCCAGGGACGAACTCACCAAGAACCAGGTGTCCCTGAC
CTGCCTGGTCAAGGGCTTTTATCCCAGCGACATCGCCGTCGAG
TGGGAGTCCAACGGACAGCCCGAGAATAACTACAAGACCACCC
CTCCTGTCCTCGACTCCGACGGCTCCTTCTTCCTGTACAGCAA
ACTGACCGTCGATAAATCTAGGTGGCAGCAGGGCAACGTGTTC
TCTTGTTCCGTGATGCATGAAGCACTGCACAACCATTATACCCA
GAAGTCTCTGAGCCTGTCCCCCGGCAAG(SEQ ID NO: 55)
The amino acid sequences of the light chains of the immunoglobulin moieties of VP101(hG1DM), VP101(hG1WT) and VP101(hG4WT) are identical, and the encoding nucleotide sequences are also identical. Amino acid sequence of the light chain of the immunoglobulin moiety in VP101(hG1DM): (214 aa) 
DIQMTQSPSSLSASVGDRVTITCSASQDISNYLNWYQQKPGKAPKV
LIYFTSSLHSGVPSRFSGSGSGTDFTLTISSLQPEDFATYYCQQYSTV
PWTFGQGTKVEIKRTVAAPSVFIFPPSDEQLKSGTASVVCLLNNFY
PREAKVQWKVDNALQSGNSQESVTEQDSKDSTYSLSSTLTLSKAD
YEKHKVYACEVTHQGLSSPVTKSFNRGEC(SEQ ID NO: 56)
Nucleotide sequence encoding the light chain of the immunoglobulin moiety in VP101(hG1DM): (642 bp)

GATATTCAGATGACTCAGAGCCCCTCCTCCCTGTCCGCCTCTGT
GGGCGACAGGGTCACCATCACATGCAGTGCTTCACAGGATATT
TCCAACTACCTGAATTGGTATCAGCAGAAGCCAGGAAAAGCAC
CCAAGGTGCTGATCTACTTCACTAGCTCCCTGCACTCAGGAGT

GCCAAGCCGGTTCAGCGGATCCGGATCTGGAACCGACTTTACT
CTGACCATTTCTAGTCTGCAGCCTGAGGATTTCGCTACATACTA
TTGCCAGCAGTATTCTACCGTGCCATGGACATTTGGCCAGGGG
ACTAAAGTCGAGATCAAGCGGACCGTGGCCGCTCCCAGTGTCT
TCATTTTTCCCCCTAGCGACGAACAGCTGAAATCCGGGACAGC
CTCTGTGGTCTGTCTGCTGAACAACTTCTACCCTAGAGAGGCA
AAAGTGCAGTGGAAGGTCGATAACGCCCTGCAGAGTGGCAATT
CACAGGAGAGCGTGACAGAACAGGACTCCAAAGATTCTACTTA
TAGTCTGTCAAGCACACTGACTCTGAGCAAGGCTGACTACGAA
AAGCATAAAGTGTATGCATGTGAGGTCACCCACCAGGGGCTGA
GCAGTCCAGTCACCAAGTCATTCAACAGAGGCGAGTGC(SEQ ID
NO: 57)

### Experimental Example 1: Detection of Promotion of Anti-CTLA4-Anti-PD-1 Bispecific Antibody in Combination with Anti-PD-1-Anti-VEGFA Bispecific Antibody on the Secretion of Cytokine IL-2 by Mixed Lvmphocvte Reaction

Two days prior to the experiment, PBMCs (from healthy donors) were thawed and cultured in a complete medium at 37 °C in a 5% carbon dioxide incubator; after 2 h, when the PBMC state was recovered, SEB (final concentration: 0.5 µg/mL) was added for stimulation for two days; on the day of the experiment, Raji-PDL1 cells were collected, centrifuged, resuspended (in 1640 + 10% FBS), counted and adjusted to a density of 200 W/mL, MMC (Mito-mycin C; final concentration: 2 µg/mL) was added, and the mixture was treated at 37 °C for 1 h in a 5% carbon dioxide incubator; PBMC after two days of SEB stimulation and Raji-PDL1 cells after MMC treatment were collected and washed twice by 1640 basic medium, the cells were resuspended by analysis medium and counted, and the cells were inoculated into a 96-well U-shaped plate (Corning, model: 3799) according to PBMC 100 thousand/well and Raji-PDL1 100 thousand/well (40 µL/well each); drugs were added according to the experimental design, blank control (Raji cell group alone and PBMC group alone) and negative control (Raji cell + PBMC group and Raji + PBMC + VEGF group) were set, and the cells were cultured for 3 days (the final volume of the system was 200 µL); wherein the final concentration of the first bispecific antibody and the second bispecific antibody when combined was 3, 30 or 300 nM; VEGF-his (prepared by Akeso Biopharma, Inc., Batch No. 20180126-1) was at a final concentration of 500 ng/mL when VEGF was added (VEGF in the experiment was VEGFA). After 3 days, the cells were centrifuged at 250× g for 5 min (Beckman centrifuge), and cell supernatants were collected, IL-2 content was detected using Dakewe kit.

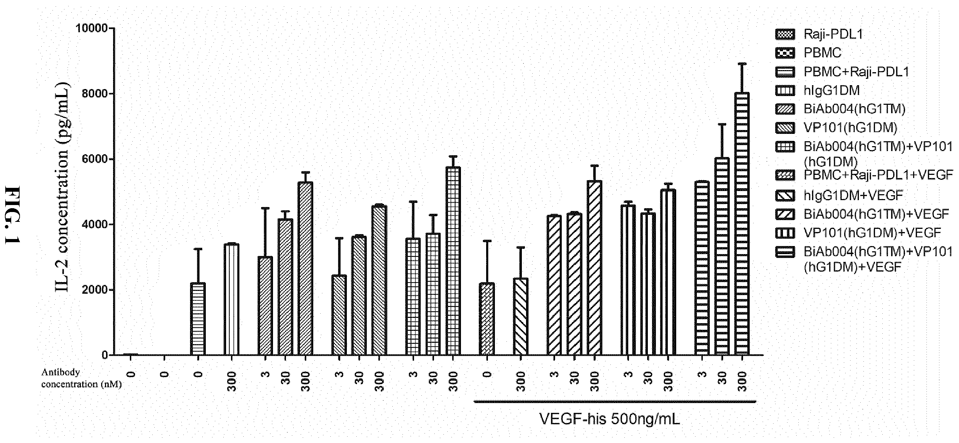

The results are shown in FIG. 1. The results show that in the mixed culture systems containing VEGF and without VEGF, the anti-CTLA4-anti-PD-1 bispecific antibody BiAb004(hG 1 TM) and the anti-PD-1-anti-VEGFA bispecific antibody VP101(hG1DM) alone or combined could significantly induce PBMC to further secrete IL-2, and the effect of the combination was superior to that of the bispecific antibody alone at the same dose when the final concentration of the antibodies was the same.

### Experimental Example 2: Detection of Promotion of Anti-CTLA4-Anti-PD-1 Bispecific Antibody in Combination with Anti-PD-1-Anti-VEGFA Bispecific Antibody on the Secretion of Cytokine bv Mixed Lvmphocvte Reaction

Normal human PBMCs were obtained by separation according to the operating instructions of Ficoll-PaqueTM Plus (GE, Cat. No. 17-1440-02). One day prior to the experiment, PBMCs were thawed and cultured in a complete medium at 37 °C in a 5% carbon dioxide incubator; on the day of the experiment, the PBMCs were collected, centrifuged, resuspended, counted, adjusted for cell density and inoculated into a 96-well U-shaped plate (Corning, 3799) according to 1 × 10⁵/well; drugs were added according to the experimental design, wherein VEGF (prepared by Akeso Biopharma, Inc., Batch No. 20210508) was incubated with the antibody for 30 min at room temperature (VEGF in the experiment was VEGFA), while negative control (PBMC + SEB + A549 cell + VEGF group, i.e., antibody-free group) and isotype control (PBMC + SEB + A549 cell + VEGF + hIgG1DM group, i.e., hIgG1DM group in the figure) were set, and were incubated with the PBMCs at 37 °C for 30 min in a 5% carbon dioxide incubator; A549 lung cancer cells (purchased from Chinese academy of sciences, Cat. No. SCSP-503) were routinely collected, centrifuged, resuspended, counted, adjusted for cell density and inoculated into the 96-well U-shaped plate described above according to 1 × 10⁴/well; SEB (final concentration: 0.1 µg/mL) (Toxin technology, Cat. No. BT202) was added at the same time, and co-cultured for 3 days (final volume of the system: 200 µL). After 3 days, the cells were centrifuged at 250× g for 5 min (Beckman centrifuge), and cell supernatants were collected, IFN-γ content was detected using Dakewe kit (Dakewe, Cat. No. 1110002).

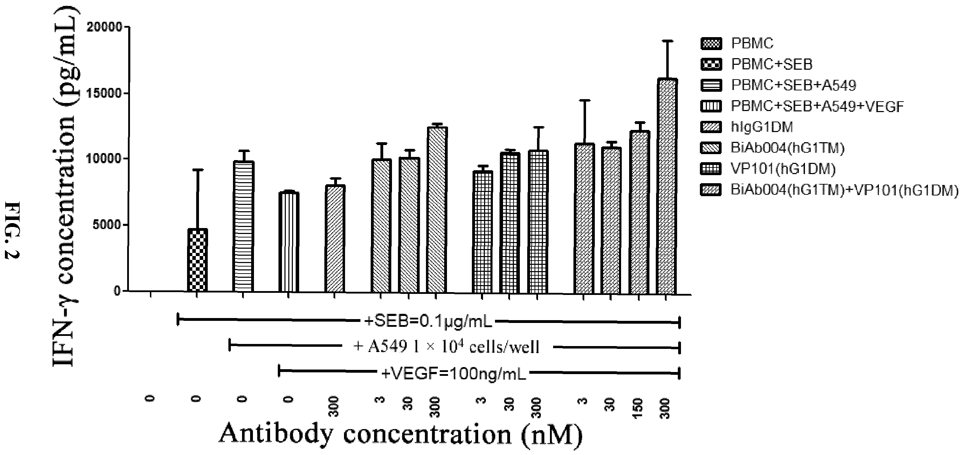

The results are shown in FIG. 2. The results show that: compared to the isotype control antibody, the anti-PD-1-anti-VEGFA bispecific antibody VP101(hG1DM) and the anti-CTLA4-anti-PD-1 bispecific antibody BiAb004(hG1TM) can effectively promote secretion of cytokine IFN-γ in a mixed lymphocyte system, and the combination group of BiAb004(hG1TM) + VP101(hG1DM) shows combined anti-tumor efficacy. The combination group is superior to the monotherapy group at the same dose in the activity of promoting secretion of cytokine IFN-γ, and the combined activity of 150 nM BiAb004(hG 1 TM) + VP101(hG1DM) is superior to that of 300 nM VP101(hG1DM) monotherapy.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

## Claims

1. A therapeutic combination comprising an effective amount of a first bispecific antibody and an effective amount of a second bispecific antibody, wherein:
(1) the first bispecific antibody comprises:
a first protein functional region targeting PD-1, and
a second protein functional region targeting CTLA4;
wherein the first protein functional region is an immunoglobulin, and the second protein functional region is a single chain antibody; or, the first protein functional region is a single chain antibody, and the second protein functional region is an immunoglobulin;
wherein,
a heavy chain variable region of the immunoglobulin comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively; a heavy chain variable region of the single chain antibody comprises HCDR1-HCDR3 having amino acid sequences set forth in SEQ ID NOs: 33-35 respectively, and a light chain variable region thereof comprises LCDR1-LCDR3 having amino acid sequences set forth in SEQ ID NOs: 36-38 respectively;
or,
a heavy chain variable region of the immunoglobulin comprises HCDR1-HCDR3 having amino acid sequences set forth in SEQ ID NOs: 33-35 respectively, and a light chain variable region thereof comprises LCDR1-LCDR3 having amino acid sequences set forth in SEQ ID NOs: 36-38 respectively; a heavy chain variable region of the single chain antibody comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively;
(2) the second bispecific antibody comprises:
a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
a heavy chain variable region of the immunoglobulin comprises HCDR1-HCDR3 having amino acid sequences set forth in SEQ ID NOs: 58-60 respectively, and a light chain variable region thereof comprises LCDR1-LCDR3 having amino acid sequences set forth in SEQ ID NOs: 61-63 respectively; a heavy chain variable region of the single chain antibody comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively;
or,
a heavy chain variable region of the immunoglobulin comprises HCDR1-HCDR3 having amino acid sequences set forth in SEQ ID NOs: 27-29 respectively, and a light chain variable region thereof comprises LCDR1-LCDR3 having amino acid sequences set forth in SEQ ID NOs: 30-32 respectively; a heavy chain variable region of the single chain antibody comprises HCDR1-HCDR3 having amino acid sequences set forth in SEQ ID NOs: 58-60 respectively, and a light chain variable region thereof comprises LCDR1-LCDR3 having amino acid sequences set forth in SEQ ID NOs: 61-63 respectively.

2. The therapeutic combination according to claim 1, wherein
in the (1), the immunoglobulin is of human IgG1 subtype; preferably, according to the EU numbering system, the heavy chain constant region of the immunoglobulin has one of the following combinations of mutations:
L234A and L235A; or
L234A and G237A; or
L235A and G237A; or
L234A, L235A and G237A;
and/or
in the (2), the immunoglobulin is of human IgG1 subtype; preferably, according to the EU numbering system, the heavy chain constant region of the immunoglobulin has one of the following combinations of mutations:
L234A and L235A; or
L234A and G237A; or
L235A and G237A; or
L234A, L235A and G237A.

3. The therapeutic combination according to any of claims 1-2, wherein in the (1):
an amino acid sequence of the heavy chain variable region of the immunoglobulin is selected from SEQ ID NO: 14 and SEQ ID NO: 18; an amino acid sequence of the light chain variable region of the immunoglobulin is selected from SEQ ID NO: 16 and SEQ ID NO: 20; an amino acid sequence of the heavy chain variable region of the single chain antibody is selected from SEQ ID NO: 2, SEQ ID NO: 6, SEQ ID NO: 10, SEQ ID NO: 41 and SEQ ID NO: 43; an amino acid sequence of the light chain variable region of the single chain antibody is selected from SEQ ID NO: 4, SEQ ID NO: 8, SEQ ID NO: 12, SEQ ID NO: 42 and SEQ ID NO: 44;
or,
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43; an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain antibody is selected from SEQ ID NO: 14 and SEQ ID NO: 18; an amino acid sequence of the light chain variable region of the single chain antibody is selected from SEQ ID NO: 16 and SEQ ID NO: 20.

4. The therapeutic combination according to any of claims 1-3, wherein the characteristics of the (1) are selected from any of the following 1) to 20):
1) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 2, and the light chain variable region of the single chain antibody has an amino acid sequence set forth in SEQ ID NO: 4;
2) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 6, and the light chain variable region of the single chain antibody has an amino acid sequence set forth in SEQ ID NO: 8;
3) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 10, and the light chain variable region of the single chain antibody has an amino acid sequence set forth in SEQ ID NO: 12;
4) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 2, and the light chain variable region of the single chain antibody has an amino acid sequence set forth in SEQ ID NO: 4;
5) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 6, and the light chain variable region of the single chain antibody has an amino acid sequence set forth in SEQ ID NO: 8;
6) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 10, and the light chain variable region of the single chain antibody has an amino acid sequence set forth in SEQ ID NO: 12;
7) the heavy chain variable region of the immunoglobulin has an amino acid sequence set forth in SEQ ID NO: 2, and the light chain variable region of the immunoglobulin has an amino acid sequence set forth in SEQ ID NO: 4; the heavy chain variable region of the single chain antibody has an amino acid sequence set forth in SEQ ID NO: 14, and the light chain variable region of the single chain antibody has an amino acid sequence set forth in SEQ ID NO: 16;
8) the heavy chain variable region of the immunoglobulin has an amino acid sequence set forth in SEQ ID NO: 2, and the light chain variable region of the immunoglobulin has an amino acid sequence set forth in SEQ ID NO: 4; the heavy chain variable region of the single chain antibody has an amino acid sequence set forth in SEQ ID NO: 18, and the light chain variable region of the single chain antibody has an amino acid sequence set forth in SEQ ID NO: 20;
9) the heavy chain variable region of the immunoglobulin has an amino acid sequence set forth in SEQ ID NO: 6, and the light chain variable region of the immunoglobulin has an amino acid sequence set forth in SEQ ID NO: 8; the heavy chain variable region of the single chain antibody has an amino acid sequence set forth in SEQ ID NO: 14, and the light chain variable region of the single chain antibody has an amino acid sequence set forth in SEQ ID NO: 16;
10) the heavy chain variable region of the immunoglobulin has an amino acid sequence set forth in SEQ ID NO: 6, and the light chain variable region of the immunoglobulin has an amino acid sequence set forth in SEQ ID NO: 8; the heavy chain variable region of the single chain antibody has an amino acid sequence set forth in SEQ ID NO: 18, and the light chain variable region of the single chain antibody has an amino acid sequence set forth in SEQ ID NO: 20;
11) the heavy chain variable region of the immunoglobulin has an amino acid sequence set forth in SEQ ID NO: 10, and the light chain variable region of the immunoglobulin has an amino acid sequence set forth in SEQ ID NO: 12; the heavy chain variable region of the single chain antibody has an amino acid sequence set forth in SEQ ID NO: 14, and the light chain variable region of the single chain antibody has an amino acid sequence set forth in SEQ ID NO: 16;
12) the heavy chain variable region of the immunoglobulin has an amino acid sequence set forth in SEQ ID NO: 10, and the light chain variable region of the immunoglobulin has an amino acid sequence set forth in SEQ ID NO: 12; the heavy chain variable region of the single chain antibody has an amino acid sequence set forth in SEQ ID NO: 18, and the light chain variable region of the single chain antibody has an amino acid sequence set forth in SEQ ID NO: 20;
13) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 41, and the light chain variable region of the single chain antibody has an amino acid sequence set forth in SEQ ID NO: 42;
14) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 43, and the light chain variable region of the single chain antibody has an amino acid sequence set forth in SEQ ID NO: 44;
15) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 41, and the light chain variable region of the single chain antibody has an amino acid sequence set forth in SEQ ID NO: 42;
16) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 43, and the light chain variable region of the single chain antibody has an amino acid sequence set forth in SEQ ID NO: 44;
17) the heavy chain variable region of the immunoglobulin has an amino acid sequence set forth in SEQ ID NO: 41, and the light chain variable region of the immunoglobulin has an amino acid sequence set forth in SEQ ID NO: 42; the heavy chain variable region of the single chain antibody has an amino acid sequence set forth in SEQ ID NO: 14, and the light chain variable region of the single chain antibody has an amino acid sequence set forth in SEQ ID NO: 16;
18) the heavy chain variable region of the immunoglobulin has an amino acid sequence set forth in SEQ ID NO: 43, and the light chain variable region of the immunoglobulin has an amino acid sequence set forth in SEQ ID NO: 44; the heavy chain variable region of the single chain antibody has an amino acid sequence set forth in SEQ ID NO: 14, and the light chain variable region of the single chain antibody has an amino acid sequence set forth in SEQ ID NO: 16;
19) the heavy chain variable region of the immunoglobulin has an amino acid sequence set forth in SEQ ID NO: 41, and the light chain variable region of the immunoglobulin has an amino acid sequence set forth in SEQ ID NO: 42; the heavy chain variable region of the single chain antibody has an amino acid sequence set forth in SEQ ID NO: 18, and the light chain variable region of the single chain antibody has an amino acid sequence set forth in SEQ ID NO: 20;
and,
20) the heavy chain variable region of the immunoglobulin has an amino acid sequence set forth in SEQ ID NO: 43, and the light chain variable region of the immunoglobulin has an amino acid sequence set forth in SEQ ID NO: 44; the heavy chain variable region of the single chain antibody has an amino acid sequence set forth in SEQ ID NO: 18, and the light chain variable region of the single chain antibody has an amino acid sequence set forth in SEQ ID NO: 20.

5. The therapeutic combination according to any of claims 1-4, wherein in the (1):
the immunoglobulin comprises a heavy chain having an amino acid sequence set forth in SEQ ID NO: 40, and a light chain having an amino acid sequence set forth in SEQ ID NO: 24.

6. The therapeutic combination according to any of claims 1-5, wherein in the (1):
the first bispecific antibody comprises:
a first protein functional region targeting PD-1, and
a second protein functional region targeting CTLA4;
the number of the first protein functional region is 1, and the number of the second protein functional region is 2;
wherein the first protein functional region is an immunoglobulin, and the second protein functional region is a single chain antibody;
the immunoglobulin comprises a heavy chain having an amino acid sequence set forth in SEQ ID NO: 40, and a light chain having an amino acid sequence set forth in SEQ ID NO: 24;
the single chain antibody comprises a heavy chain variable region having an amino acid sequence set forth in SEQ ID NO: 43, and a light chain variable region having an amino acid sequence set forth in SEQ ID NO: 44;
the single chain antibody is linked to the C terminus or the N terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

7. The therapeutic combination according to any of claims 1-6, wherein in the (2):
an amino acid sequence of the heavy chain variable region of the immunoglobulin is set forth in SEQ ID NO: 45, and an amino acid sequence of the light chain variable region of the immunoglobulin is set forth in SEQ ID NO: 47; an amino acid sequence of the heavy chain variable region of the single chain antibody is selected from SEQ ID NO: 14 and SEQ ID NO: 18, and an amino acid sequence of the light chain variable region of the single chain antibody is selected from SEQ ID NO: 16, SEQ ID NO: 20 and SEQ ID NO: 49;
or,
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16, SEQ ID NO: 20 and SEQ ID NO: 49; an amino acid sequence of the heavy chain variable region of the single chain antibody is set forth in SEQ ID NO: 45, and an amino acid sequence of the light chain variable region of the single chain antibody is set forth in SEQ ID NO: 47.

8. The therapeutic combination according to any of claims 1-7, wherein the characteristics of the (2) are selected from any of the following 1) to 12):
1) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 16;
2) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 20;
3) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 14, and the light chain variable region of the single chain antibody has an amino acid sequence set forth in SEQ ID NO: 49;
4) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 16;
5) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 20;
6) the heavy chain variable region of the immunoglobulin has an amino acid sequence set forth in SEQ ID NO: 45, and the light chain variable region of the immunoglobulin has an amino acid sequence set forth in SEQ ID NO: 47; the heavy chain variable region of the single chain antibody has an amino acid sequence set forth in SEQ ID NO: 18, and the light chain variable region of the single chain antibody has an amino acid sequence set forth in SEQ ID NO: 49;
7) the heavy chain variable region of the immunoglobulin has an amino acid sequence set forth in SEQ ID NO: 14, and the light chain variable region of the immunoglobulin has an amino acid sequence set forth in SEQ ID NO: 16; the heavy chain variable region of the single chain antibody has an amino acid sequence set forth in SEQ ID NO: 45, and the light chain variable region of the single chain antibody has an amino acid sequence set forth in SEQ ID NO: 47;
8) the heavy chain variable region of the immunoglobulin has an amino acid sequence set forth in SEQ ID NO: 14, and the light chain variable region of the immunoglobulin has an amino acid sequence set forth in SEQ ID NO: 20; the heavy chain variable region of the single chain antibody has an amino acid sequence set forth in SEQ ID NO: 45, and the light chain variable region of the single chain antibody has an amino acid sequence set forth in SEQ ID NO: 47;
9) the heavy chain variable region of the immunoglobulin has an amino acid sequence set forth in SEQ ID NO: 14, and the light chain variable region of the immunoglobulin has an amino acid sequence set forth in SEQ ID NO: 49; the heavy chain variable region of the single chain antibody has an amino acid sequence set forth in SEQ ID NO: 45, and the light chain variable region of the single chain antibody has an amino acid sequence set forth in SEQ ID NO: 47;
10) the heavy chain variable region of the immunoglobulin has an amino acid sequence set forth in SEQ ID NO: 18, and the light chain variable region of the immunoglobulin has an amino acid sequence set forth in SEQ ID NO: 16; the heavy chain variable region of the single chain antibody has an amino acid sequence set forth in SEQ ID NO: 45, and the light chain variable region of the single chain antibody has an amino acid sequence set forth in SEQ ID NO: 47;
11) the heavy chain variable region of the immunoglobulin has an amino acid sequence set forth in SEQ ID NO: 18, and the light chain variable region of the immunoglobulin has an amino acid sequence set forth in SEQ ID NO: 20; the heavy chain variable region of the single chain antibody has an amino acid sequence set forth in SEQ ID NO: 45, and the light chain variable region of the single chain antibody has an amino acid sequence set forth in SEQ ID NO: 47; and
12) the heavy chain variable region of the immunoglobulin has an amino acid sequence set forth in SEQ ID NO: 18, and the light chain variable region of the immunoglobulin has an amino acid sequence set forth in SEQ ID NO: 49; the heavy chain variable region of the single chain antibody has an amino acid sequence set forth in SEQ ID NO: 45, and the light chain variable region of the single chain antibody has an amino acid sequence set forth in SEQ ID NO: 47.

9. The therapeutic combination according to any of claims 1-8, wherein in the (2):
the immunoglobulin comprises a heavy chain having an amino acid sequence set forth in SEQ ID NO: 54, and a light chain having an amino acid sequence set forth in SEQ ID NO: 56.

10. The therapeutic combination according to any of claims 1-9, wherein in the (2):
the second bispecific antibody comprises:
a first protein functional region targeting PD-1, and
a second protein functional region targeting VEGFA;
the number of the first protein functional region is 1, and the number of the second protein functional region is 2;
wherein the first protein functional region is an immunoglobulin, and the second protein functional region is a single chain antibody;
the immunoglobulin comprises a heavy chain having an amino acid sequence set forth in SEQ ID NO: 54, and a light chain having an amino acid sequence set forth in SEQ ID NO: 56;
the single chain antibody comprises a heavy chain variable region having an amino acid sequence set forth in SEQ ID NO: 18, and a light chain variable region having an amino acid sequence set forth in SEQ ID NO: 49;
the single chain antibody is linked to the C terminus or the N terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different; preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

11. The therapeutic combination according to any of claims 1-10, wherein in the (1) and/or the (2):
the first protein functional region is linked to the second protein functional region either directly or via a linker fragment; and/or the heavy chain variable region of the single chain antibody is linked to the light chain variable region of the single chain antibody either directly or via a linker fragment.

12. The therapeutic combination according to claim 11, wherein in the (1) and/or the (2):
the linker fragment is (GGGGS)n, wherein n is a positive integer, preferably, n is 1, 2, 3, 4, 5 or 6.

13. The therapeutic combination according to any of claims 1-12, wherein in the (1) and/or the (2):
the numbers of the first protein functional region and the second protein functional region are each independently 1, 2 or more.

14. The therapeutic combination according to any of claims 1-13, wherein in the (1) and/or the (2):
the single chain antibody is linked to the C terminus or the N terminus of the heavy chain of the immunoglobulin.

15. The therapeutic combination according to any of claims 1-14, wherein the first bispecific antibody and the second bispecific antibody are mixed together, and the therapeutic combination is a pharmaceutical composition.

16. The therapeutic combination according to any of claims 1-15, wherein the first bispecific antibody and the second bispecific antibody are in separate packages, and the therapeutic combination is a combination product.

17. The therapeutic combination according to any of claims 1-16, wherein the mass ratio of the first bispecific antibody to the second bispecific antibody is (10:1) to (1:5), preferably (5:1) to (1:3) or (3:1) to (1:2), and more preferably 2:1.

18. The therapeutic combination according to any of claims 1-17, wherein the unit dose of the first bispecific antibody and the second bispecific antibody is independently 100 mg to 2500 mg, 100 mg to 2000 mg, 100 mg to 1500 mg, 100 mg to 1200 mg, 100 mg to 1000 mg, 200 mg to 800 mg, 200 mg to 500 mg, 300 mg to 600 mg, 400 mg to 500 mg, or 450 mg, based on the mass of the first bispecific antibody or the second bispecific antibody.

19. The therapeutic combination according to any of claims 1-18, wherein the therapeutic combination further comprises an effective amount of an anti-tumor chemotherapeutic drug, such as an alkylating agent, an antimetabolite, an anti-tumor antibiotic, a plant-based anticancer agent, a hormone, or an immunological agent.

20. The therapeutic combination according to any of claims 1-19, wherein the therapeutic combination further comprises one or more pharmaceutically acceptable adjuvants; preferably, further comprises a package insert.

21. Use of the therapeutic combination according to any of claims 1-20 for preparing a medicament for the treatment or prevention of a tumor; preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

22. The therapeutic combination according to any of claims 1-20 for use in the treatment or prevention of a tumor;
preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer; preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

23. A method for treating or preventing a tumor, comprising administering to a subject in need thereof an effective amount of the therapeutic combination according to any of claims 1-20; wherein
preferably, the tumor is selected from one or more of pancreatic cancer, breast cancer, ovarian cancer, colorectal cancer, cervical tumor, plasma cell cancer, head and neck cancer, brain cancer, throat cancer, nasopharyngeal cancer, oesophageal cancer, oesophageal squamous cancer, thyroid cancer, mesothelioma, lung cancer, breast cancer, liver cancer, gastric cancer, biliary tract cancer, kidney cancer, fallopian tube cancer, endometrial cancer, cervical cancer, bladder cancer, urothelial carcinoma, prostate cancer, testicular cancer, skin cancer, melanoma, myeloma, plasma cell cancer, vulval cancer, leukaemia, lymphoma, bone cancer, and osteosarcoma;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the lymphoma is Hodgkin's lymphoma or non-Hodgkin's lymphoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

24. The method according to claim 23, wherein the first bispecific antibody and the second bispecific antibody are administered independently before or after surgery and/or before or after radiotherapy.

25. The method according to any of claims 23-24, wherein
the unit dose of the first bispecific antibody and the second bispecific antibody is independently 0.1-100 mg per kg body weight, preferably 5-50 mg or 5-15 mg per kg body weight;
the first bispecific antibody and the second bispecific antibody are independently administered once every half day, 1 day, 2 days, 3 days, 4 days, 5 days, 6 days, 10 days, 1 week, 2 weeks or 3 weeks;
and/or
the route of administration is intravenous drip infusion, intravenous injection, subcutaneous injection or intraperitoneal injection.
